# EUROPEAN PATENT SPECIFICATION

(11) **EP 3 162 894 B1**
(45) Date of publication and mention of the grant of the patent: **10.01.2024**
(21) Application number: 16193811.3
(22) Date of filing: 19.05.2011
(51) Int. Cl.: C12N 9/52

(54) **METHODS FOR THE MANUFACTURE OF PROTEOLYTICALLY PROCESSED POLYPEPTIDES**
VERFAHREN ZUR HERSTELLUNG PROTEOLYTISCH VERARBEITETER POLYPEPTIDE
PROCÉDÉ POUR LA FABRICATION DE POLYPEPTIDES TRAITÉS PROTÉOLITIQUEMENT

(43) Date of publication of application: 03.05.2017
(62) Divisional of application: 11004152.2
(73) Proprietor: Ipsen Bioinnovation Limited, Abingdon, Oxfordshire OX14 4RY (GB)
(72) Inventor: RUMMEL, Andreas, 30171 Hannover (DE)
(74) Representative: MacLean, Martin Robert

(56) References cited:
- US-A1- 2010 022 751
- KOZAKI S ET AL: "Activation of Clostridium botulinum type B and E derivative toxins with lysine-specific proteases", FEMS MICROBIOLOGY LETTERS, WILEY-BLACKWELL PUBLISHING LTD, GB, vol. 27, no. 2, 1 May 1985 (1985-05-01), pages 149-154, XP023919407, ISSN: 0378-1097, DOI: 10.1111/J.1574-6968.1985.TB00658.X [retrieved on 1985-05-01]
- TIA ESTEY ET AL: "Evaluation of chemical degradation of a trivalent recombinant protein vaccine against botulinum neurotoxin by LysC peptide mapping and MALDI-TOF mass spectrometry", JOURNAL OF PHARMACEUTICAL SCIENCES, vol. 98, no. 9, 1 September 2009 (2009-09-01), pages 2994-3012, XP55040203, ISSN: 0022-3549, DOI: 10.1002/jps.21543
- GIMENEZ J A ET AL: "Botulinum neurotoxin type E fragmented with endoproteinase Lys-C reveals the site trypsin nicks and homology with tetanus neurotoxin", BIOCHIMIE, MASSON, PARIS, FR, vol. 72, no. 4, 1 April 1990 (1990-04-01), pages 213-217, XP025458768, ISSN: 0300-9084, DOI: 10.1016/0300-9084(90)90075-R [retrieved on 1990-04-01]

## Description

A novel proteolytically active polypeptide and various uses of the polypeptide in screening and manufacturing methods are described herein.

Clostridium botulinum and Clostridium tetani produce highly potent neurotoxins, i.e. botulinum neurotoxins (BoNTs) and tetanus neurotoxin (TeNT), respectively. These clostridial neurotoxins (CNTs) specifically bind to neuronal cells and disrupt neurotransmitter release. Clostridium botulinum secretes seven antigenically distinct serotypes designated A to G of the botulinum neurotoxin (BoNT). All serotypes together with the related tetanus neurotoxin (TeNT) secreted by Clostridium tetani, are Zn²⁺-endoproteases that block synaptic exocytosis by cleaving proteins involved in the formation of the SNARE complex controlling cellular membrane fusion. CNTs cause the flaccid muscular paralysis seen in botulism and tetanus. In addition, CNT activity has been shown to affect glandular secretion. These physiological effects of CNTs on muscular and glandular activity are increasingly used in various therapeutic and cosmetic applications. Botulinum neurotoxin of serotype A (BoNT/A) was approved for human use in the United States in 1989 for the treatment of strabism, blepharospasm, and other disorders. It is commercially available as a Botulinum toxin A protein preparation, for example, under the tradename BOTOX (Allergan Inc.) and under the tradename DYSPORT (Ipsen Ltd). For therapeutic application, a complex comprising the neurotoxin and additional bacterial proteins is injected directly into the muscle to be treated. At physiological pH, the toxin is released from the protein complex (Eisele et al. 2011, Toxicon 57(4):555-65.) and the desired pharmacological effect takes place. An improved BoNT/A preparation being free of complexing proteins is available under the tradenames XEOMIN or Bocouture (Merz Pharmaceuticals GmbH, Frankfurt/Germany). The effect of BoNT is only temporary, which is the reason why repeated administration of BoNT is usually required to maintain a therapeutic affect.

Each CNT is initially synthesised as an inactive single chain polypeptide. In the case of BoNT, the neurotoxin polypeptide has a molecular weight of approximately 150 kDa. The posttranslational processing of this single chain polypeptide involves limited proteolysis in an exposed region called loop (see table 1) and the formation of a nearby disulfide bridge. Active di-chain neurotoxin consists of two cleavage products resulting from the proteolytic hydrolysis of the single chain precursor polypeptide: an N-terminal light chain of approx. 50 kDa and a heavy chain of approx. 100 kDa linked by a disulfide bond. CNTs structurally consist of three domains, i.e. the catalytic light chain, the heavy chain encompassing the translocation domain (N-terminal half) and the receptor binding domain (C-terminal half) (cf. Krieglstein 1990, Eur J Biochem 188: 39; Krieglstein 1991, Eur J Biochem 202: 41; Krieglstein 1994, J Protein Chem 13: 49; Lacy et al., 1998, Nat. Struct. Biol. 5(10):898-902). Depending on the number of cleavage sites present in the single chain between the amino acid residues forming the catalytic domain and the amino acid residues forming the translocation domain, endopeptidase activity can give rise to two large cleavage products, i.e. the light and the heavy chain and, in addition, characteristic short peptides representing the former loop region, bridging in the single chain of the neurotoxin what is to become the light and the heavy chain (cf. table 1, below).

The purification of the CNTs from the fermentation solution is a particular challenge, since the neurotoxins are contained therein as a mixture of unprocessed, partially processed and fully processed polypeptides, all of which have very similar biochemical and physical properties. Partially processed neurotoxins are typically generated, if the endoproteolytic activity has hydrolysed the peptide bond between the light chain and the loop, while the peptide bond between the loop and the N-terminus of the heavy chain is still intact. Moreover, partially processed neurotoxin can also be created if the endoproteolytic activity has released the loop peptide from the heavy chain, while the peptide bond between the loop peptide and the C-terminus of the light chain has not yet been hydrolysed. Depending on the conditions of fermentation and the type of neurotoxin, the fully processed polypeptide which is devoid of the loop peptide can be contaminated significantly, with between 5% to 90% partially processed or unprocessed polypeptide. Yet in some cases, the neurotoxin is mainly unprocessed and, prior to therapeutic use, needs to be treated with an endopeptidase in order to become biologically active.

The prior art describes various attempts to treat clostridial neurotoxins with heterologous proteases in order to reduce the amount of unprocessed or partially processed precursor protein. The protease most widely used for activation of clostridial neurotoxins, Trypsin, while being useful for activating clostridial neurotoxins of serotypes B (BoNT/B) and E (BoNT/E) (DasGupta & Sugiyama 1972, Biochem. Biophys. Res. Commun. 48: 108-112; Kozaki et al., 1974, Infect. Immun. 10: 750-756) appears to produce secondary products, presumably by proteolytic action near the C-terminus of the heavy subunit of BoNT/A and, thus, appears to destroy toxin binding to its cellular receptor (Shone et al., 1985, Eur. J. Bioch. 151: 75-82). More specific cleavage products are theoretically expected from endogenous proteases, isolated from the native host, such as C. botulinum producing BoNT/A. Accordingly, various attempts have been made to isolate from the native host cell the endogenous protease involved in proteolytic activation of clostridial neurotoxins. Dekleva & DasGupta (Dekleva & DasGupta, 1989, Biochem. Biophys. Res. Commun. 162: 767-772) purified from cultures of C. botulinum producing BoNT/A a fraction capable of proteolytically cleaving BoNT/A into a heavy and a light subunit. Later studies of the same authors further characterised the endogenous protease isolated from C. botulinum (Dekleva & DasGupta, 1990, J. Bact. 172: 2498-2503) and revealed a 62kDa protein, composed of a 15.5 kDa polypeptide and a 48 kDa polypeptide. However, the observation of considerable fragmentation of CNTs after limited exposure to the 62kDa protein of Dekleva & DasGupta suggests that the isolated protease may not be the unidentified proteolytic enzyme responsible for the activation of CNTs in clostridial cell cultures and during infection. In fact, others have recently suggested that Clostripain, also designated clostridiopeptidase B (Mitchel & Harrington, 1968, JBC 243: 4683-4692), might be involved in the specific activation of CNTs (Sebaihia et al., 2007, Genome Res. 17(7): 1082-1092; WO2009/014854). Interestingly, the structure and substrate specificity of this enzyme are reminiscent of those of the secreted alpha-clostripain from *Clostridium histolyticum* (Dargatz et al. 1993), a homolog (74% amino acid identity) of which is present in *C. botulinum* (CBO1920). The *C*. *histolyticum* alpha-clostripain is a cysteine endopeptidase with strict specificity for the arginyl bond. It is synthesized as an inactive prepro-enzyme that undergoes an autocatalytic cleavage to generate 15.4- and 43-kDa polypeptides, which associate to form a heterodimeric active enzyme (Dargatz et al. 1993). Both the *C*. *histolyticum* alphaclostripain and the *C. botulinum* 62-kDa protease require a reducing agent and calcium for full activity and are susceptible to the same protease inhibitors. These data strongly suggest that the *C. botulinum* ortholog of alpha-clostripain (CBO1920) is the endogenous protease responsible for the proteolytic nicking of the neurotoxin of *C. botulinum.* A gene encoding clostripain (CPE0846) is also present in *C. perfringens*, and has been found to be positively regulated by the two-component system VirRlVirS (Shimizu et al. 2002b). Kozaki et al (1985), FEMS Microbiology Letters, 27, 2, 149-154 describes activation of *Clostridium botulinum* type B and E derivative toxins with lysine-specific proteases. Estey et al (2009), Journal of Pharmaceutical Sciences, 98, 9, 2994-3012 describes evaluation of chemical degradation of a trivalent recombinant protein vaccine against botulinum neurotoxin by LysC peptide mapping and MALDI-TOF mass spectrometry. US 2010/022751 A1 describes recombinant toxin fragments.

Till this day, however, further conclusive experimental evidence is missing and a protease capable of efficiently converting the single chain precursor CNTs into the authentic mature cleavage products, i.e. the di-chain neurotoxin, is still not available in the art.

Means and methods for reducing the amount of unprocessed and/or partially processed neurotoxin polypeptides and thereby improving the quality of neurotoxin preparations are highly desirable but not yet available. Thus, the technical problem underlying the present invention may be seen as the provision of means and methods for improving the manufacture of neurotoxin polypeptides by complying with the aforementioned needs. The technical problem is solved by the embodiments characterised in the claims and herein below.

A proteolytically active polypeptide which comprises a polypeptide sequence having at least 50 % sequence identity with the sequence of SEQ ID NO: 1 is described herein. A proteolytically active polypeptide consisting of a polypeptide sequence having at least 50 % sequence identity with the sequence of SEQ ID NO: 1 is described herein. A proteolytically active polypeptide consisting of a polypeptide sequence as shown in SEQ ID NO: 1 is described herein.

The term "proteolytically active polypeptide" as used herein refers to the catalytic function of the polypeptide of the present invention and means that the polypeptide of the present invention is capable of hydrolysing a peptide bond. A "proteolytically active polypeptide" may refer to a polypeptide that is capable of hydrolysing a polypeptide comprising an amino acid sequence selected from any one of SEQ ID NOs: 4 to 25. The term "proteolytically inactive polypeptide" as used herein refers to the catalytic function of the polypeptide of the present invention and means that the polypeptide of the present invention is incapable of hydrolysing a peptide bond.

The skilled person can determine whether a polypeptide according to the sequence definition mentioned herein is a polypeptide described herein, by testing the proteolytic activity of said polypeptide. An assay or test system for determining proteolytic activity comprises contacting a polypeptide which comprises a polypeptide sequence having at least 50% sequence identity with the sequence of SEQ ID NO: 1 with a test substrate. A test substrate is typically a polypeptide which is known to be cleavable by a polypeptide described herein. A test substrate which is a CNT such as BoNT or a fragment thereof is described herein. The test substrate can be e.g. uncleaved/ unprocessed BoNT, designated herein as "scBoNT" and can be e.g. of serotype A, B, C1, D, E, F or G (e.g. "scBoNTIA", "scBoNT/B" etc.) or the test substrate can be tetanus neurotoxin. Alternatively, the test substrate can be a fragment of a clostridial neurotoxin, said fragment comprising an amino acid sequence selected from any one of SEQ ID NOs: 4 to 25. The fragment can be a polypeptide of 50 or more amino acid residues or a peptide of up to 49 amino acid residues. As used throughout the present specification, the term "polypeptide" refers to molecules with 50 or more amino acid residues, whereas the term "peptide" refers to molecules with 2 to 49 amino acid residues. The test substrate may be a soluble neurotoxin fragment called LH_{N} comprising the light chain polypeptide, the exposed loop peptide region and the N-terminal half of the heavy chain polypeptide, the translocation domain H_{N}. The test substrate may be or may comprise a peptide selected from any one of SEQ ID NOs: 4 to 25 (cf. table 1). The test substrate may be a chimeric neurotoxin comprising amino acid residues derived from two or more serotypes.

An assay for determining the proteolytic activity would typically comprise a step of determining the degree of conversion of the test substrate into its cleavage product(s). The observation of one or more cleavage product(s) generated after contacting the polypeptide with the test substrate or the observation of an increase in the amount of cleavage product(s) is indicative of proteolytic activity of the polypeptide. Said step of determining may involve comparing substrate and cleavage product(s). Said comparing may involve determining the amount of substrate and/or the amount of one or more cleavage product(s) and may also involve calculating the ratio of substrate and cleavage product(s). In addition, the assay for determining the proteolytic activity may comprise a step of comparing a test sample with a reference sample, wherein the reference sample typically comprises (a) a polypeptide which comprises a polypeptide sequence having at least 50% sequence identity with the sequence of SEQ ID NO: 1 and which is known to be proteolytically active and (b) a test substrate known to be cleavable by the polypeptide of (a). The assay for determining the proteolytic activity may comprise separating substrate and cleavage product(s) by electrophoresis or by column chromatography and, optionally, a spectrometric analysis. It may be convenient to label the test substrate with one or more labels in order to more easily detect decrease of test substrate and/or increase of product(s). The term "label", as used herein, means detectable marker and includes e.g. radioactive label, antibody, fluorescent label. The amount of test substrate and/or cleavage product may be determined e.g. by methods of autoradiography or spectrometry, including methods based on energy resonance transfer between at least two labels. Alternatively, immunological methods such as western blot or ELISA may be used for detection. A preferred assay for determining the proteolytic activity of a polypeptide taught herein is described herein below in the Examples. A polypeptide may be proteolytically active, if more than 20%, preferably more than 95% of test substrate is converted into the cleavage products such as the light chain and the heavy chain in 120 min at 37°C using a buffer selected from 100 mM Tris-HCl, pH 8.0 or PBS (50 mM Na₂HPO₄, 150 mM NaCl, pH 7.4). The same conditions apply, if the test substrate is not a full length neurotoxin but, instead, e.g. a fragment of the full length neurotoxin or a derivative of the neurotoxin. It is apparent that the cleavage products will differ in this case. However, the skilled person can quantify the corresponding cleavage products. Typically, 100 ng of proteolytically active polypeptide and a molar ratio of 1:100 with regard to the substrate may be used in the assay. A sample may be taken at intervals in order to follow the catalytic activity over time. The assay may be modified, e.g. by using multiple amounts of the proteolytically active polypeptide.

SEQ ID NO: 2 shows the polypeptide sequence of a proteolytically inactive polypeptide derived from a Clostridium botulinum strain ATCC 3502, GenBank accession No: "CAL82988.1", having an amino acid length of 581 residues. SEQ ID NO: 1 shows a proteolytically active derivative of SEQ ID NO: 2, lacking amino acid residues 1 to 248 of SEQ ID NO: 2.

The term "polypeptide which comprises a polypeptide sequence having at least 50% sequence identity with the sequence of SEQ ID NO: 1" refers to a polypeptide which has at least 50% sequence identity with the sequence of SEQ ID NO: 1. In addition, the term refers to a polypeptide which comprises a polypeptide sequence having at least 50% sequence identity with the sequence of SEQ ID NO: 1. Said polypeptide may have additional amino acids, for example at an internal position or N- or C-terminal to the sequence shown in SEQ ID NO: 1 or at an internal position or N- or C-terminal to a amino acid sequence which is at least 50% identical with sequence of SEQ ID NO: 1, wherein a methionine may be present at the N-terminus of the polypeptide. In addition, the term refers to a polypeptide lacking one or more amino acid residues, for example at an internal position or at the N- or C-terminus of the sequence shown in SEQ ID NO: 1 or at an internal position or the N- or C-terminus of a sequence which is at least 50% identical in sequence to SEQ ID NO: 1.

The term "sequence identity" as used herein refers to determination of the identity between a reference amino acid sequence and a query sequence wherein the sequences are aligned so that the highest order match is obtained, and which can be calculated using published techniques or methods codified in computer programs such as, for example, BLASTP, BLASTN, FASTA (Altschul 1990, J Mol Biol 215: 403). The percent identity values are in one aspect calculated over the entire amino acid sequence. Sequence identity may be calculated over a sequence length of up to 50aa residues, up to 100aa, up to 150aa, up to 250aa, 300aa, 350aa, 400aa, 450aa, 500aa, or 550aa residues. Sequence identity may be calculated over at least 50aa residues, at least 100aa, at least 150aa or at least 250aa residues. Sequence identity may be determined over the entire length of SEQ ID NO: 1 or 2, i.e. over a length of 333aa or 581aa, respectively. A series of programs based on a variety of algorithms is available to the skilled worker for comparing different sequences. In this context, the algorithms of Needleman and Wunsch or Smith and Waterman give particularly reliable results. To carry out the sequence alignments and calculate the sequence identity values recited herein, the commercially available program DNASTAR Lasergene MegAlign version 7.1.0 based on the algorithm Clustal W was used over the entire sequence region with the following settings: Pairwise Alignment parameters: Gap Penalty: 10.00, Gap Length Penalty: 0.10, Protein weight matrix Gonnet 250, which, unless otherwise specified, shall always be used as standard settings for sequence alignments.

The term "at least 50% sequence identity" as used herein means at least 50%, at least 55%, at least 60%, at least 65%, at least 70%, at least 75%, at least 80%, at least 85%, at least 90%, at least 95% or 100%.

The proteolytically active polypeptide described herein may have the same number of amino acids as the reference polypeptide sequence as shown in SEQ ID NO: 1. Also comprised are polypeptides having additional or less amino acid residues. A proteolytically active polypeptide which is or comprises a truncation mutant of SEQ ID NO:1 or 2 or of a polypeptide having at least 50% sequence identity with the sequence of SEQ ID NO: 1 or 2 is described herein. The truncation mutant of SEQ ID NO: 2 may for example lack one or more amino acid residues N-terminal to amino acid position 249. A truncation mutant may be an N- or C-terminal truncation mutant and/or an internal truncation mutant that is proteolytically active. A truncation mutant of SEQ ID NO:2 lacking amino acid positions 1 to 248 of SEQ ID NO: 2 is described herein. A truncation mutant of SEQ ID NO: 2 which is a C-terminal truncation mutant is described herein. A truncation mutant lacking up to 1, 2, 3, 4, 5, 6, 7, 8, 9, 10, 15, 20, 50, 100, 150 or up to 170 consecutive amino acid residues is described herein. A proteolytically active polypeptide which has an amino acid length of at least 200aa residues, of at least 250aa residues, of at least 300aa residues or of at least 333aa residues is described herein. A proteolytically active polypeptide which has up to 333aa residues, up to 350aa residues, up to 573 residues, up to 581aa residues, up to 592aa residues, up to 600aa or up to 617aa residues is described herein.

A proteolytically active polypeptide is described herein which encompasses a polypeptide comprising additional amino acid residues at the N- or C-terminus and/or at an internal position of the polypeptide chain of SEQ ID NO: 1 or a of a polypeptide sequence having at least 50% sequence identity with the sequence of SEQ ID NO: 1. These additional amino acid residues may comprise up to 5, up to 10 or even up to 200, 300 or up to 400 consecutive amino acid residues. The additional amino acid residues may function as an inhibitor of the proteolytic activity. The additional amino acid residues can be removed by a protease. Additional residues inhibiting the proteolytic activity of the polypeptide may be excluded. The additional amino acid residues may be flanked by one or more protease cleavage sites. The additional amino acid sequence may function as a detectable tag and/or allows binding to a solid support

A polypeptide chain of SEQ ID NO: 1 or a of a polypeptide sequence having at least 50% sequence identity with the sequence of SEQ ID NO: 1 which is modified by exchanging one or more amino acid residues is described herein. The term "exchanging", as used herein, means replacing an amino acid with a different amino acid. For example, up to 1aa, 2aa, 3aa, 4aa, 5aa, 6aa, 7aa, 8aa, 9aa, 10aa, 15aa, 20aa or up to 50aa may be replaced within the polypeptide sequence. The exchanges may involve conservative or non-conservative amino acid changes, aiming e.g. at increasing or decreasing substrate binding or proteolytic activity of the polypeptide described herein.

A proteolytically active polypeptide encompassing a polypeptide that is capable of hydrolysing a substrate into two or more native cleavage product(s) is described herein. A polypeptide which hydrolyses the substrate into two or more cleavage products which differ from the native cleavage products is described herein. The term "native cleavage products" or "native products" as used herein refers to products, which are identical in amino acid sequence when compared to products generated from the same substrate in wildtype cell cultures, from which the substrate originates. A cleavage product which is the di-chain neurotoxin of a botulinum neurotoxin or of tetanus neurotoxin is described herein, a di-chain neurotoxin which is a neurotoxin isolated from C. botulinum of serotype A, B, C1, D, E, F or G is described herein. In yet another disclosure, said di-chain neurotoxin is a native di-chain neurotoxin.

**Table 1 shows the precursor, the native di-chain neurotoxin of TeNT and of BoNT/A-G and identifies the exposed loop comprising the amino acid sequence cleaved by the polypeptide described herein.**

| **Toxin** | **exposed loop** | **LC** | **H_{N}** | **H_{CN}** | **H_{CC}** |
|---|---|---|---|---|---|
| BoNT/A1 | SEQ ID NO: 4 | M1-K438 | A449-N872 | I873-S1092 | N1093-L1296 |
| BoNT/A2 | SEQ ID NO: 5 | M1-K438 | A449-N872 | I873-S1092 | N1093-L1296 |
| BoNT/A3 | SEQ ID NO: 6 | M1-K434 | A445-N868 | I869-S1088 | N1089-L1292 |
| BoNT/A3 | SEQ ID NO: 7 | M1-K434 | A445-N868 | I869-S1088 | N1089-L1292 |
| BoNT/A4 | SEQ ID NO: 8 | M1-K438 | A449-N872 | I873-S1092 | N1093-L1296 |
| BoNT/A5 | SEQ ID NO: 9 | M1-K438 | A449-N872 | I873-S1092 | N1093-L1296 |
| BoNT/A6 | SEQ ID NO: 5 | M1-K438 | A449-N872 | I873-S1093 | N1094-L1297 |
| BoNT/A7 | SEQ ID NO: 10 | M1-K438 | A449-N872 | I873-S1092 | N1093-L1296 |
| BoNT/B1 | SEQ ID NO: 11 | M1-K441 | A442-I860 | L861-S1079 | Y1080-E1291 |
| BoNT/B2 | SEQ ID NO: 12 | M1-R441 | A442-1860 | L861-S1079 | Y1080-E1291 |
| BoNT/B3 | SEQ ID NO: 12 | M1-R441 | A442-I860 | L861-S1079 | Y1080-E1291 |
| BoNT/B4bv | SEQ ID NO: 11 | M1-K441 | A442-I860 | L861-S1079 | Y1080-E1291 |
| BoNT/B5nP | SEQ ID NO: 13 | M1-K441 | V442-I860 | L861-S1079 | Y1080-E1291 |
| BoNT/B6 | SEQ ID NO: 11 | M1-K441 | A442-I860 | L861-S1079 | Y1080-E1291 |
| BoNT/C1 | SEQ ID NO: 14 | M1-R444 | T450-I868 | N869-L1092 | Q1093-E1291 |
| BoNT/CD | SEQ ID NO: 14 | M1-R444 | T450-1868 | N869-Q1083 | I1084-E1280 |
| BoNT/D | SEQ ID NO: 15 | M1-K442 | D446-I864 | N865-QI079 | 11080-E1276 |
| BoNT/DC | SEQ ID NO: 16 | M1-R442 | D446-I864 | N865-L1088 | Q1089-E1285 |
| BoNT/E1-E5 | SEQ ID NO: 17 | M1-K419 | S424-I847 | K848-P1067 | N1068-K1252 |
| BoNT/E6 | SEQ ID NO: 18 | M1-K419 | S424-I847 | K848-P1067 | N1068-K1252 |
| BoNT/F1 | SEQ ID NO: 19 | M1-R435 | A440-I866 | K867-P1085 | D1086-N1278 |
| BoNT/F2 | SEQ ID NO: 20 | M1-R435 | Q440-I866 | K867-P1088 | D1089-E1280 |
| BoNT/F3 | SEQ ID NO: 20 | M1-R435 | Q440-I866 | K867-P1088 | D1089-E1279 |
| BoNT/F4 | SEQ ID NO: 21 | M1-R435 | A440-I866 | K867-P1085 | D1086-E1277 |
| BoNT/F5 | SEQ ID NO: 22 | M1-K434 | P440-1863 | K864-P1085 | D1086-E1277 |
| BoNT/F6 | SEQ ID NO: 19 | M1-R435 | A440-I866 | K867-P1088 | D1089-E1275 |
| BoNT/F7 | SEQ ID NO: 23 | M1-K427 | N432-1857 | I858-P1076 | D1077-E1268 |
| BoNT/G | SEQ ID NO: 24 | M1-K442 | S447-1865 | S866-S1086 | S1087-E1297 |
| TeNT | SEQ ID NO: 25 | M1-R449 | T456-K883 | S884-L1109 | S1110-D1315 |

It is to be understood that the definitions and explanations of the terms made above and below apply mutatis mutandis for all aspects described in this specification unless otherwise indicated.

The proteolytically active polypeptide described herein is suitable for various applications. A commercially relevant application is its use in the production of therapeutic neurotoxins, such as those isolated from C. botulinum. At present, the cell cultures of C. botulinum used for the preparation of commercially available preparations of Botulinum neurotoxin are contaminated with significant amounts of partially processed and/or unprocessed neurotoxin, both of which negatively impair, i.e. reduce the specific activity of these pharmaceutical compositions. Using the proteolytically active or activated polypeptide described herein for example after lysis of C. botulinum, it will now be possible to treat compositions comprising unprocessed and/or partially processed neurotoxin and, thus, convert these contaminants into fully processed neurotoxin. In consequence, commercial products can be provided with an increased specific activity of the neurotoxin, wherein the total amount of bacterial protein can be reduced, further reducing the patients risk of antibody formation.

A nucleic acid molecule comprising a nucleic acid sequence encoding the polypeptide of the present invention and, optionally, regulatory elements is described herein. The term "regulatory elements" as used herein refers to regulatory elements of gene expression, including transcription and translation, and includes elements such as tata box, promotor, enhancer, ribosome binding site, Shine-Dalgamo-sequence, IRES-region, polyadenylation signal, terminal capping structure, and the like. Said regulatory element may comprise one or more heterologous regulatory elements or one or more homologous regulatory elements. A "homologous regulatory element" is a regulatory element of a wildtype cell, from which the nucleic acid molecule of the present invention is derived, which is involved in the regulation of gene expression of the nucleic acid molecule or the polypeptide in said wildtype cell. Nucleic acid molecules comprising heterologous regulatory elements are described herein. The term "heterologous regulatory element" is a regulatory element which is not involved in the regulation of gene expression of the nucleic acid molecule or the polypeptide in said wildtype cell. Also regulatory elements for inducible expression, such as inducible promoters, are encompassed. The nucleic acid molecule can be, for example, hnRNA, mRNA, RNA, DNA, PNA, LNA, and/or modified nucleic acid molecules. The nucleic acid molecule can be circular, linear, integrated into a genome or episomal. Also concatemers coding for fusion proteins comprising three, four, five, six, seven, eight, nine or ten polypeptides of the present invention are encompassed. Moreover, the nucleic acid molecule may contain sequences encoding signal sequences for intracellular transport such as signals for transport into an intracellular compartment or for transport across the cellular membrane.

A vector is taught comprising a nucleic acid molecule according to the nucleic acid molecule described herein. A vector may be suitable for in vitro and/or in vivo expression of the polypeptide described herein. The vector can be a vector for transient and/or stable gene expression. A vector furthermore comprising regulatory elements and/or selection markers is described herein. A vector of virus origin, phage origin, or of bacterial origin is described herein.

A cell is described herein comprising the nucleic acid molecule or the vector taught herein. The term "cell" as used herein, encompasses prokaryotic and/or eukaryotic cells suitable to express said nucleic acid molecule or said vector and in particular the polypeptide described herein. Said cell may be a host cell not expressing the polypeptide described herein or a homolog thereof. The term "homolog" as used herein refers to a polypeptide comprising a polypeptide sequence having at least 50% sequence identity with the sequence of SEQ ID NO: 1. However, also described herein are cells, in particular wildtype cells, expressing the polypeptide described herein or a homolog thereof. The cell described herein may be selected from C. botulinum, C. butyricum, C. baratii and C. tetani. The cell may be C. botulinum of serotype A, B or F. The cell may be the Hall strain (ATCC 3502) of C. botulinum. The cell may be the BoNT/A producing strain ATCC 19397 also known as NCTC 4587 and NCTC 7272 of C. botulinum. The cell may be the BoNT/A producing strain NCTC 2916 of C. botulinum. Said cell may be the BoNT/A2 producing strains Kyoto-F and Mauritius / NCTC 9837 of C. botulinum. Said cell may be the BoNT/A3 producing strain A254 Loch Maree / NCTC 2012 of C. botulinum. Said cell may be the BoNT/A4 and B producing strain CDC657 of C. botulinum. Said cell may be the BoNT/A5 and B3' producing strain H04402 065 of C. *botulinum.* Said cell may be the BoNT/B1 producing strain Okra / NCTC 7273 of C. botulinum. Said cell may be the BoNT/B and F producing strain CDC4013 / NCTC 12265 of C. botulinum. Said cell may be the BoNT/F1 producing strain Langeland / NCTC 10281 of C. botulinum. Said cell may be Clostridium sporogenes, Clostridium perfringens, Clostridium acetobutylicum, B. cereus, B. thuringiensis, B. mycoidis, B. thermoproteolyticus, B. anthracis, B. megaterium, B. subtilis, E.coli, or a yeast cell. The polypeptide described herein may be modified inside the cell (i.e. glycosylated, phosphorylated, processed by proteases, etc.). Modification also includes the addition of non-proteinaceous co-factors including metal-ions. Cells comprising the proteolytically inactive polypeptide described above, any intermediate polypeptide product, as well as the final proteolytically active polypeptide disclosed herein are described herein. Also described herein are cells comprising an inducer of expression of the polypeptide described herein. Such an inducer of expression may be a nucleic acid molecule or a polypeptide or a chemical entity, including a small chemical entity, having the effect of increasing the amount or activity of proteolytically active polypeptide described herein in cell cultures or lysates thereof. The inducer of expression may e.g. increase transcription or translation of a nucleic acid molecule encoding the polypeptide described herein. Alternatively, the inducer of expression may be a compound capable of activating the proteolytically inactive polypeptide SEQ ID NO:2 or a polypeptide comprising a polypeptide sequence having at least 50% sequence identity with the sequence of SEQ ID NO: 2. A cell comprising an inducer that is a proteolytically active polypeptide capable of removing inhibitory amino acid residues from the N-terminus of said polypeptide is described herein. The inducer may be, for example, expressed by recombinant means known to the person skilled in the art. Alternatively, the inducer may be isolated from a cell, e.g. a clostridial cell.

Taught herein is the use of the nucleic acid molecule described herein for the manufacture of the proteolytically active polypeptide described herein.

A method for the manufacture of a proteolytically active polypeptide, comprising the steps of: (a) chemically synthesising or translating from a nucleotide sequence a polypeptide, comprising a polypeptide sequence having at least 50% sequence identity with the sequence of SEQ ID NO: 1; and (b) purifying the polypeptide of step (a.) is described herein.

The term "chemically synthesising" means synthesising polypeptides by chemical means. Such methods are reviewed for example in Nilsson et al., Ann. Rev. Biophys. Biomol. Struct. 2005. 34:91-118. The term "purifying the polypeptide" means removing from a mixture comprising the polypeptide described herein compounds other than said polypeptide. The term also means removing the polypeptide described herein from a mixture comprising compounds other than said polypeptide. The term may mean separating the proteolytically active polypeptide from its proteolytically inactive precursor.

The nucleic acid may be translated in a cell or in a cell free system. Various systems for cell free translation are available to the skilled person. Translation in a cell-free protein translation system comprising rabbit reticulocyte lysate, wheat germ lysate, E. coli lysate, or other cellular lysates, for example lysates generated from C. botulinum and the like is described herein. Also described herein is translating the polypeptide described herein from the nucleotide sequence or the vector described herein. Transcription may be regulated or controlled by one or more heterologous regulatory elements or by homologous regulatory elements. Also described herein is the translation in a wildtype cell, i.e. a cell isolated from nature, such as any known isolate of C. botulinum, C. butyricum, C. baratii, and C. tetani. Said cell may be C. botulinum Hall strain (ATCC 3502). Various standard means and methods are available to the skilled person for bringing a nucleic acid molecule or a vector into the cell and for expressing the polypeptide described herein as recombinant protein in a cell. Moreover, the skilled person knows many standard techniques for isolating polypeptides from cells or cell lysates or from cell free expression systems (e.g. Recombinant DNA Principles and Methodologies, J. Green, Marcel Dekker Inc., 1998; The Condensed Protocols: From Molecular Cloning: A Laboratory Manual, Sambrook et al, Cold Spring Harbor Laboratory, 2006; Molecular Cloning: A Laboratory Manual, Sambrook et al., Cold Spring Harbor Laboratory, 2000). Any of these means and methods may be used in the methods described herein.

The first polypeptide described herein may be translated from a nucleic acid molecule encoding the proteolytically active polypeptide. SEQ ID NO: 26 is an example of such a nucleic acid molecule. Alternatively, said nucleic acid molecule may encode a precursor polypeptide which is proteolytically inactive but which can be converted into the proteolytically active polypeptide described herein. SEQ ID NO: 27 is an example of such a nucleic acid molecule. The proteolytically inactive precursor is also designated "inactive BoNTHydrolase", abbreviated iBH. This proteolytically inactive polypeptide may e.g. be activated during or after translation or by contacting, for example, said proteolytically inactive polypeptide with a protease capable of removing inactivating amino acid residues at the N-terminus of the proteolytically inactive polypeptide. An example of a proteolytically inactive polypeptide is the polypeptide represented by SEQ ID NO: 2. Another example is a polypeptide comprising a polypeptide sequence having at least 50% sequence identity with the sequence of SEQ ID NO: 2. The term "inactivating amino acid residues at the N-terminus" in one aspect relates to the first 248aa residues of said polypeptide. This term may refer to a fragment of up to 1 0aa, 50aa, 1 00aa, 150aa, 200aa, 250aa residues of said polypeptide. Any of these polypeptides are useful in the method for the manufacture of a proteolytically active polypeptide described herein. A protease capable of removing inactivating amino acid residues from the N-terminus of this polypeptide is isolated e.g. from Clostridium botulinum, Clostridium butyricum, Clostridium baratii, and Clostridium tetani is described herein. A protease capable of removing said inactivating amino acid is provided by providing a fractionated or non-fractionated lysate of said cells is described herein. Inactivating amino acid residues may be removed by contacting the proteolytically inactive polypeptide with said lysate and incubating until the proteolytically inactive polypeptide is transformed into the proteolytically active polypeptide.

A method in which the polypeptide is translated in a cell is described herein. The cell may be a prokaryotic or eukaryotic cell. A cell selected from E. coli, B. subtilis or yeast is described herein. Also described herein is the translation of the polypeptide described herein in a wildtype cell, i.e. a cell isolated from nature, such as any known isolate of Clostridium botulinum, Clostridium butyricum, Clostridium baratii, and Clostridium tetani. Said cell may be C. botulinum Hall strain (ATCC 3502). Said cell may be the cell described herein above.

Translation products obtained by the method may be purified by various means, all of which are known to the person skilled in the art (e.g. Recombinant DNA Principles and Methodologies, J. Green, Marcel Dekker Inc., 1998; The Condensed Protocols: From Molecular Cloning: A Laboratory Manual, Sambrook et al, Cold Spring Harbor Laboratory, 2006; Molecular Cloning: A Laboratory Manual, Sambrook et al., Cold Spring Harbor Laboratory, 2000). Typical methods of purifying the polypeptide described herein may involve spinning of cell lysate, ammonium sulphate precipitation of proteins, resuspension of proteins, centrifugation of resuspended proteins, ion exchange chromatography, size exclusion chromatography, hydrophobic interaction chromatography and the like. Several combinations of such steps, in differing order, may be useful for purifying the polypeptide. A method for purifying the polypeptide is described in the Examples.

In one description, the step of purifying comprises binding the polypeptide to a solid support. The term "solid support" refers to a matrix encompassing e.g. silica, crosslinked dextran, crosslinked polyacrylamid or crosslinked agarose and the like. Also included are in particular polypeptides, glass, polystyrene, polypropylene, polyethylene, polyethylene glycol (PEG), dextran, nylon, amylases, natural and modified celluloses, polyacrylamides, gabbros, and magnetite. A solid support may be a polysaccharide matrix selected from the group consisting of sepharose, sephadex, agarose, sephacell, micro-cellulose, and alginate-beads. Said solid support can consist of glass-beads, and/or polypeptide matrices.

In one description, the solid support is linked to the antibody described herein. The term "linked" means in one description stably linked or stably associated. In another description, linked includes interactions such as indirect or direct, non-reversible or reversible, physical and chemical, electrostatic, and/or covalent bonds. In a description, the antibody is covalently linked, either directly or via a linker molecule, to the solid support. The antibody may be bound to said solid support via a linker, including small molecular compounds and peptide (or polypeptide) linker molecules. The solid support can have virtually any possible structural configuration or arrangement as long as the coupled antibody is capable of binding to its antigen. Thus, the matrix or solid support may be spherical, as in a bead, or cylindrical, as in the inside surface of a test tube, or the external surface of a rod. Alternatively, the surface may be irregular or flat such as a sheet or test strip.

Said antibody linked to the solid support can be used e.g. in a manufacturing method or in a diagnostic method. Said manufacturing method may comprise a step of affinity chromatography, wherein said affinity chromatography is based on an antibody linked to a solid support. Said antibody may be an antibody specifically binding to the proteolytically active polypeptide described herein. Said antibody may be an antibody specifically binding to the proteolytically inactive polypeptide described herein.

A method for manufacturing the proteolytically active polypeptide comprising purifying the polypeptide from a mixture containing additional components is described herein. Purification may be based on e.g. polarity, electrical charge and size. Therefore, the method may comprise one or more steps of separation selected from the group consisting of: normal-phase HPLC, reversed-phase HPLC, hydrophilic-interaction chromatography (HILIC), hydrophobic-interaction chromatography (HIC), ion-exchange chromatography (IEC) including anion-exchange chromatography and cation-exchange chromatography, size-exclusion chromatography (SEC), gel-permeation chromatography (GPC).

A purification comprising the steps of: (a) separation by anion exchange chromatography; (b) separation by size exclusion chromatography; (c) separation by hydrophobic interaction chromatography; and (d) separation by size exclusion chromatography is described herein.

One or more fractions collected from a chromatography column can be concentrated e.g. by precipitation or ultrafiltration.

A composition comprising the proteolytically active polypeptide is described herein. Using the method disclosed herein, it is possible to manufacture proteolytically active polypeptide, which is substantially free of proteolytically inactive polypeptide. In other words, the method provides a proteolytically active polypeptide and a composition comprising no substantial contamination with inactive precursor protein of the polypeptide. A composition is deemed to contain no substantial contamination or to be substantially free of proteolytically inactive precursor polypeptide if, by using a western blot based detection method, less than 5% of proteolytically inactive precursor can be detected, wherein said 5% refer to the amount of proteolytically inactive precursor in relation to the sum of proteolytically active and inactive polypeptide. Said composition may be substantially pure and comprise at least 50% proteolytically active polypeptide, wherein said 50% refers to the amount of proteolytically active precursor in relation to the total amount of protein contained in the composition. Said substantially pure composition may comprise at least 75%, 80%, 90% or at least 98% proteolytically active polypeptide.

A polypeptide which is obtainable from the method for the manufacture of a proteolytically active polypeptide as described herein above and as illustrated in the Examples is described herein. A proteolytically active polypeptide which is a proteolytically active polypeptide with the polypeptide sequence of SEQ ID NO: 1 is described herein. A proteolytically active polypeptide having at least 50% sequence identity with the sequence of SEQ ID NO: 1 is described herein. A proteolytically active polypeptide which comprises a polypeptide sequence having at least 50% sequence identity with the sequence of SEQ ID NO: 1 is described herein. The term "polypeptide which is obtainable", as used herein refers in one aspect to a polypeptide that is translated from the nucleic acid described herein. The polypeptide can subsequently undergo posttranslational modification such as acylation, alkylation, amidation, amino acid addition, amino acid deletion, glycosylation, oxidation, S-glutathionylation, phosphorylation, sulphatation, proteolytic processing and the like. Moreover, the polypeptide may bind to a metal ion such as Li⁺, Na⁺, K⁺, Ag⁺, Cs⁺, Mg²⁺, Ca²⁺, Co²⁺, Ni²⁺, Mn²⁺, Cu²⁺ or Zn²⁺. Preferably, said metal ion is Zn²⁺, Mn²⁺ or Co²⁺.

Taught herein is an antibody specifically binding to the polypeptide described herein. The term "antibody" as used herein encompasses a monoclonal antibody, a polyclonal antibody, a single chain antibody, a human, humanized, primatized, or chimerized antibody, a bispecific antibody, a synthetic antibody, chemically or enzymatically modified derivatives, a fragment of any of said antibodies or aptamers consisting of naturally occurring and/or chemically modified nucleic acids. Fragments of said antibodies include F(ab')2, F(ab), Fv or scFv fragments or chemically or enzymatically modified derivatives of any of these fragments.

In one description, the antibody shall specifically bind to the proteolytically active polypeptide described herein or its proteolytically inactive precursor. An antibody which is specific for the proteolytically active polypeptide cross-reacts the proteolytically inactive polypeptide described herein. An antibody capable of discriminating between the proteolytically active polypeptide and its inactive precursor is described herein. In another description, the epitope for which said antibody is specific is located in an amino acid region that is present in the proteolytically inactive polypeptide but not in the proteolytically active polypeptide. For example, said epitope can be an epitope of a polypeptide region consisting of amino acid residues 1 to 248 of a polypeptide comprising the polypeptide sequence having at least 50% sequence identity with the sequence of SEQ ID NO: 2.

An epitope formed by amino acid residues located N-terminal to amino acid 249 of a polypeptide comprising the polypeptide sequence having at least 50% sequence identity with the sequence of SEQ ID NO: 2 is described herein. Said epitope may be removed from the proteolytically inactive polypeptide described herein by proteolytic processing.

In one description, the epitope for which the antibody is specific is an epitope located at the N-terminus of a polypeptide comprising the polypeptide sequence having at least 50% sequence identity with the sequence of SEQ ID NO: 1. The term "N-terminus", as used in this description, refers to a region of the polypeptide comprising the N-terminal 50 amino acid residues of said polypeptide sequence, preferably the N-terminal 25 amino acid residues of said polypeptide sequence. In a particular description, the term refers to the N-terminal 14 amino acid residues. The term "epitope" as used herein relates to the antigenic determinant which is recognised by the antibody described herein. In one description, the epitope is a linear epitope, in another description the epitope is a conformational epitope. In a particular description, the antigenic determinant consists of a peptide having the amino acid sequence of the N-terminus of the proteolytically active polypeptide described herein, wherein said peptide can have an amino acid length of 7 to 14, preferably of 8, 9, 10, 11, 12, 13 or 14 amino acid residues.

The term "specifically binds" or "specifically binding to" in one description means that the antibody does not cross-react to a significant extent with other epitopes either on the polypeptide described herein or on other polypeptides in general. Epitope specificity is an important characteristic of the antibody. Specificity of the antibody with respect to the proteolytically active versus proteolytically inactive polypeptide shall be, in an aspect, at least 95%, at least 96%, at least 97%, at least 98%, at least 99%. Specific binding can be tested by various well known techniques including, e.g., competition studies. Another important characteristic is the sensitivity of the antibody. Sensitivity shall be, in one description, such that at least 70%, at least 80%, at least 90%, at least 95% of the epitope comprised by a sample is bound. Sensitivity can be tested by well known techniques. Those skilled in the art will be able to determine operative and optimal assay conditions for each determination by employing routine experimentation. Conventional techniques for binding studies include radioimmunoassay, ELISA, equilibrium dialysis, isothermal microcalorimetry, BIACORE^{®} assays (surface plasmon reasonance, SPR) or other surface adsorption methods. The BIACORE^{®} SPR system measures the antibody-antigen interaction. SPR response reflects a change in mass concentration at the detector surface as analytes bind or dissociate. Based on SPR, real-time BIACORE^{®} measurements monitor interactions directly as they occur, see BIAapplications Handbook, version AB (reprinted 1998), BIACORE^{®} code No: BR-1001-86; BIAtechnology Handbook, version AB (reprinted 1998), BIACORE^{®} code No: BR-1001-84. The binding properties such as sensitivity of an antibody of the present invention may, in principle, be determined by binding studies using an immobilised antigen (the ligand) presented on a sensor surface. The antibody to be tested (the analyte) will be provided in the mobile phase, i.e. in a solution. In some cases, the antigen is attached indirectly to the surface through binding to another immobilised molecule which is referred as the capturing molecule. When the antibody is injected in a discrete pulse across the surface with the immobilised antigens, essentially three phases can be subdivided: (i) Association of antibody with the antigen during sample injection; (ii) Equilibrium or steady state during sample injection, where the rate of antibody binding is balanced by dissociation from the antibody-antigen complex; (iii) Dissociation of antibody from the surface during buffer flow. It will be understood that such an assay can alternatively be performed with immobilised antibodies to be investigated and an antigen containing solution as the mobile phase. The association and dissociation phases provide information on the kinetics of analyte-ligand interaction (kₐ and k_{d}, the rates of complex formation and dissociation, k_{d}/kₐ=K_{D}). The equilibrium phase provides information on the affinity of the analyte-ligand interaction (K_{D}). The antibody may have a K_{D} of less than 0.5 µM, in another description less than 0.05 µM and, in another description, less than 0.02 µM.

The antibody as referred to herein can be manufactured by using methods which are described, e.g., in Harlow and Lane, 1988 (Harlow and Lane, "Antibodies, A Laboratory Manual", CSH Press, Cold Spring Harbor, 1988). Monoclonal antibodies can be prepared by the techniques originally described in Kohler & Milstein, 1975 (Kohler & Milstein 1975, Nature 256: 495) and Galfre & Milstein, 1981 (Galfre & Milstein 1981, Meth Enzymol 73: 3). Said techniques comprise the fusion of mouse myeloma cells to spleen cells derived from immunized mammals. Antibodies can be further improved by techniques well known in the art. For example, surface plasmon resonance as employed in the BIACORE^{®} system can be used to increase the efficiency of phage antibodies which bind to the aforementioned epitope within polypeptide of the present invention (cf. Schier et al., 1996, Human Antibodies Hybridomas 7: 97; Malmborg et al., 1995, J. Immunol Methods 183: 7).

An antibody produced by using a peptide comprising or consisting of the aforementioned epitope is described herein. The peptide can be produced e.g. synthetically or by recombinant expression. Alternatively, the antibody can be produced by applying natural occurring proteolytically active or inactive polypeptide described herein. In the latter case, it is to be understood that the resulting antibodies shall be further tested for specificity with respect to the polypeptide described herein. A monoclonal antibody produced by using a polypeptide described herein which can be treated by a detergent in order to make the epitope immunologically available is described herein. However, it will be understood that in a case where the antibody shall be directed against a conformational epitope, no such detergent treatment shall be carried out. Immune-stimulation agents such as keyhole limpet hemocyanin (KLH) may be also applied in such process, especially when using a synthetic peptide.

The antibody can be used, for example, for affinity chromatography, immunoprecipitation, and immunolocalization of the polypeptide of the present invention as well as for the monitoring of the presence of said polypeptide in samples or in recombinant organisms. Moreover, the antibody may be used in a detection method or in a diagnostic method. In a particular description, the antibody is used in Western Blot or ELISA. In addition, the antibody can be used in therapeutic applications. In particular, the antibody can be used for inhibiting the activity of the proteolytically active polypeptide described herein. Therefore, the antibody may also have various therapeutic applications described herein below.

The use of the proteolytically active polypeptide described herein in a method for proteolytically processing a polypeptide is described herein. A method for the manufacture of a proteolytically processed polypeptide, comprising the step of contacting: (a) a first polypeptide, said first polypeptide being the polypeptide described herein, with (b) a second polypeptide, said second polypeptide being susceptible to proteolysis by said first polypeptide, wherein said contacting results in proteolytic processing of said second polypeptide into at least two cleavage products is described herein.

The use of Lys-N or Lys-C and arginyl endopeptidase (endoproteinase Arg-C, LeR) from Lysobacter enzymogenes (ATCC 29487) (Wright DS, Graham LD, Jennings PA. Biochim Biophys Acta. 1998 Dec 22;1443(3):369-74) is described herein. The use of plasmin and/or omptin (OmpT), a membrane-bound serine protease that cleaves at (Arg/Lys)-(Arg/Lys) motifs (K. Sugimura and T. Nishihara. J. Bacteriol. 170 (1988), pp. 5625-5632) in a method for proteolytically processing CNT such as BoNT/A is described herein. In one aspect, the present invention relates to a method for the manufacture of a proteolytically processed polypeptide, comprising the step of contacting: (a) a first polypeptide, said first polypeptide being Lys-C or Lys-N, with (b) a second polypeptide, said second polypeptide being susceptible to proteolysis by said first polypeptide, wherein said contacting results in proteolytic processing of said second polypeptide into at least two cleavage products, and wherein the second polypeptide is a single chain BoNT/F and wherein said first polypeptide hydrolyses the single-chain BoNT/F to produce an active di-chain BoNT/F. The term "Lys-C" refers to the 33 kDa serine endoproteinase Lys-C from Lysobacter enzymogenes (Lysyl endopeptidase, LeK, Genbank ace. Q7M135) that specifically cleaves peptide bonds C-terminally to lysine or a homolog thereof having at least 60% sequence identity. The term "Lys-N" refers to the metalloendopeptidase Lys-N isolated from Grifola frondosa and Pleurotus ostreatus (Nonaka T et al., 1997, J Biol Chem. 272:30032-30039; Nonaka T et al., 1998, J Biochem. 124:157-162; Hori T et al., 2001, Acta Crystallogr D Biol Crystallogr. 57:361-368). Also encompassed by the term are homologs of said protease having at least 60% sequence identity.

A method used, for example, for manufacturing proteolytically processed neurotoxin (CNT) or botulinum neurotoxin (BONT) is described herein. The term "BoNT", as used throughout, means Botulinum neurotoxin and refers to neurotoxin obtainable from C. botulinum such as BoNT of serotype A, B, C1, D, E, F or G. Also encompassed by the term "CNT" and "BoNT" is recombinant and modified neurotoxin comprising one or more modifications including chemical modification or genetic modification. The term "genetic modification" means deletion, substitution or addition of one or more amino acid residues. Using the method of the present invention, it is now possible to obtain neurotoxin compositions with significantly less contamination by unprocessed or partially processed neurotoxin, since those contaminants are efficiently processed into di-chain neurotoxin. In one aspect, the di-chain (BoNT/F) is a native di-chain (BoNT/F), wherein the C-terminus of the light chain and the N-terminus of the heavy chain are identical to the corresponding fully processed di-chain (BoNT/F) isolated from wildtype clostridia.

The term "contacting" as used herein refers to bringing at least two different compounds in physical proximity as to allow physical and/or chemical interaction of said compounds. In accordance with the method of this invention, the said two different compounds are, in an aspect, the first and the second polypeptide which are comprised by the solution. Contacting is carried out under conditions and for a time being sufficient to allow interaction of the first and second polypeptide. The term "proteolytically processed polypeptide" as used herein refers in one aspect to a polypeptide, the polypeptide chain of which has been hydrolysed or cleaved at one or more peptide bonds. In another aspect, the term refers to a polypeptide that has been proteolytically cleaved by an endoproteinase or endopeptidase. In another aspect, the term refers to a polypeptide which has been cleaved to a degree of at least 50%. In another aspect, said proteolytically processed polypeptide is the second polypeptide. In another aspect, at least 60%, 70%, 80%, 90% or 95% are proteolytically processed.

The term "second polypeptide", as used herein, refers to the substrate of said first polypeptide. The term "being susceptible to proteolysis" refers to a feature or requirement of the second polypeptide and is used herein meaning that the second polypeptide is proteolytically cleavable by said first polypeptide. In other words, the term "being susceptible to proteolysis" means that the second polypeptide comprises a protease recognition and cleavage site allowing it to function as a substrate of the first polypeptide. The "second polypeptide" is a substrate of the first polypeptide and is proteolytically processed into two or more cleavage products. Using the assay described herein above, the skilled person can test whether a given polypeptide is a substrate of the first polypeptide and, thus, a "second polypeptide" according to present invention's definition. The term "at least two cleavage products" includes, for example, up to two, three, four, five and up to six cleavage products.

This method can be used, for example, for preparing a pharmaceutical composition comprising clostridial neurotoxin or for generating polypeptide fragments used in a method of mass spectrometry. The first polypeptide and the second polypeptide can be contacted at various steps in the manufacturing process of proteolytically processed polypeptide. In one aspect, the step of contacting the first polypeptide and the second polypeptide is within a cell. In a particular aspect of this embodiment, the first and the second polypeptide are expressed in said cell.

In another aspect, said step of contacting is in a cell lysate or in a purified cell lysate. This aspect encompasses adding the first polypeptide to the lysate or the purified lysate. The first polypeptide can be added at various steps during purification of the second polypeptide from the cell lysate. For example, the first polypeptide can be added prior to or after: protein precipitation, ion exchange chromatography, hydrophobic interaction chromatography and/or size exclusion chromatography. Moreover, also encompassed is the addition of the first polypeptide to a pharmaceutical composition. In this aspect, the polypeptide of the present invention is used e.g. for proteolytically cleaving the second polypeptide e.g. for activating a second polypeptide which is a therapeutic agent contained in the pharmaceutical composition. Also envisaged is the administration of the first polypeptide to a subject, in order to proteolytically process a second polypeptide in the subject. Administration also includes coadministration of the first and the second polypeptide. Also encompassed by this method is a step of incubation at conditions and for a time sufficient to cleave the second polypeptide. In one aspect, the conditions can comprise adding a buffer selected from the group consisting of 100 mM Tris-HCl, pH 8.0 or PBS (50 mM Na₂HPO₄, 150 mM NaCl, pH 7.4). Preferred buffer conditions include 100 mM Tris-HCl, pH 8.0. The "time sufficient to cleave" can be determined using the assay described herein above. In one aspect, said "time sufficient to cleave" depends on the degree of cleavage that the proteolytically processed polypeptide or a composition comprising it should have. In one aspect, the method comprises a step of incubating the first and the second polypeptide for at least 30min, 60min, 120min or at least 240min. In another aspect, the first and second polypeptide are incubated for up to 30min, 60min, 120min, 240min, 480min or up to 600min. In another aspect, the method comprises a step of incubating the first and the second polypeptide at 4°C or at 37°C. In another aspect, the method comprises a step of incubating for up to 1 h, up to 2h, 4h, 6h, 10b or up to 16h.

A polypeptide chain of said second polypeptide comprising a sequence selected from any one of SEQ ID NOs: 4 to 25 is described herein. A polypeptide chain of said second polypeptide comprising a sequence selected from any one of SEQ ID NOs: 4 to 25 and wherein the second polypeptide is cleaved C-terminal to a basic amino acid residue within said sequence of any one of SEQ ID NOs: 4 to 25 is described herein. Said sequences represent amino acid sequences of known substrates of the proteolytically active polypeptide of the present disclosure. As shown herein, said substrates are cleaved C-terminal to a basic amino acid residue contained in the sequence, compare Table 1, column LC and H_{N}. A second polypeptide comprising a sequence selected from SEQ ID NO: 4 to 10 is described herein. A second polypeptide which is BoNT/A or a derivative thereof, including e.g. the polypeptide of SEQ ID NO: 3 and derivatives thereof is described herein. The term "derivative" as used with respect to this and other aspects of the invention, comprises amino acid mutations such as addition, substitution, deletion or truncation of one or more amino acid residues.

A second polypeptide comprising a derivative of any one of SEQ ID NOs: 4 to 25, or of SEQ ID NO: 3, wherein said derivative has one or more point mutation and/or one or more additional amino acid residues is described herein. In another aspect, said derivative has up to 1, up to 2, up to 3, up to 4, up to 5, up to 6, up to 7, up to 8, up to 9, up to 10, up to 15 point mutations. By using the activity assay for determining protease activity, as described herein, the skilled person can determine whether a given derivative is processed by the proteolytically active polypeptide described herein. A derivative containing a point mutation changing a basic amino acid residue into a non-basic amino acid residue is described herein. A derivative having at least 50% sequence identity with any one of SEQ ID NOs: 4 to 25 is described herein. A derivative or a polypeptide comprising the derivative is a substrate of the first polypeptide and is proteolytically cleavable by the first polypeptide is described herein. A typical example is a derivative of SEQ ID NO: 3 comprising e.g. one or more point mutations in the light or heavy chain.

A second polypeptide comprising (a) a polypeptide sequence having at least 30% sequence identity with the sequence of SEQ ID NO: 3 [(BoNT/A of ATCC 3502, Genbank acc. AAA23262 )]; or (b) a polypeptide sequence selected from the group consisting of Tetanus neurotoxin, protein of the coagulation cascade Factor X or Prothrombin (Factor II), digestive enzymes of the pancreas like trypsin, chymotrypsin, pepsin, papain is described herein. At least 30% means at least 30%, at least 40%, at least 50%, at least 85%. The sequence identity of said second polypeptide sequence having at least 50% sequence identity with the sequence of SEQ ID NO: 3 may be determined based on amino acid position 420 to 466 of SEQ ID NO: 3, in another example, said sequence identity may be determined based on any one of SEQ ID NOs: 4 to 25. A second polypeptide which comprises a polypeptide sequence which has e.g. at least 30% sequence identity to the polypeptide sequence found between amino acid positions 420 to 466 of SEQ ID NO: 3 or at least 30% sequence identity to the polypeptide sequence of any one of SEQ ID NOs: 4 to 25 is described herein. A polypeptide according to this definition is, e.g. obtainable from C. botulinum, C. tetani or C. sporogenes. A second polypeptide which may be, for example, a naturally occurring neurotoxin such as BoNT/A, B, C1, D, E, F or G or a derivative thereof comprising one or more amino acid mutations such as addition, substitution, deletion or truncation of one or more amino acid residues is described herein. Encompassed are e.g. derivatives lacking e.g. the native neurotoxin H_{C} domain or parts thereof or derivatives with other amino acid residues replacing the neurotoxin H_{C} domain as well as derivatives with an additional light chain or another proteinaceous cargo molecule fused N-terminally to the light chain of BoNT.

In another aspect, the second polypeptide may contain additional amino acid residues at the N- or C-terminus or at an internal position. The additional amino acid residues may be flanked by one or more protease cleavage sites. In another aspect, the additional amino acid sequence functions as a detectable tag and/or allows binding to a solid support. An example is a his tag or a GST tag. Another example is the amino acid sequence VPPTPGSAWSHPQFEK containing the Streptag, preferably added to the C-terminus.

A second polypeptide which is a polypeptide comprising a polypeptide sequence as shown in GenBank no: CBZ04958.1, YP_002805603.1, ZP_02994746.1, YP_001788403.1, YP_001782718.1, ZP_02616437.1, ZP_02614241.1, YP_001392361.1, YP_001255575.1 or a homolog thereof having at least 50% sequence identity is described herein.

In another aspect, the biological activity of said second polypeptide is modulated by the proteolytic cleavage. It is well known to the skilled person, that the function of many polypeptides can be modulated by proteolytic processing. "Modulated" as used herein means increased or decreased, activated or inactivated. For example, the biological activity of many clostridial neurotoxins is increased or triggered by proteolytically processing a single chain neurotoxin into a di-chain neurotoxin, wherein the di-chain neurotoxin is composed of a light and a heavy polypeptide chain, which are covalently linked through a disulfide-bridge. The biological activity of the neurotoxin encompasses at least three separate activities: the first activity is a "proteolytic activity" residing in the light chain of the neurotoxin and is responsible for hydrolysing the peptide bond of one or more polypeptides involved in the regulation of cellular membrane fusion. A second activity is a "translocation activity", residing at the N-terminal end of the heavy chain of the processed neurotoxin and is involved in the transport of the light chain across the lysosomal membrane and into the cytoplasm. A third activity is a "receptor binding activity", residing at the C-terminal end of the heavy chain of the processed neurotoxin and involved in binding and uptake of the neurotoxin to a target cell. In a preferred aspect, the term biological activity as used herein means proteolytic activity. In a more preferred aspect, the term means increased proteolytic activity.

Biological activity of clostridial neurotoxin can be measured by various tests, all of which are known to the person skilled in the art. These tests allow determining one or more of the activities mentioned above. For example, the mouse LD₅₀ assay or the ex vivo mouse phrenic nerve hemidiaphragm (MPN) assay as described by Pearce et al., 1994 (Pearce LB, Borodic GE, First ER, MacCallum RD (1994), Toxicol Appl Pharmacol 128: 69-77) and Habermann et al., 1980 (Habermann E, Dreyer F, Bigalke H. (1980), Naunyn Schmiedebergs Arch Pharmacol. 311:33-40) allow determining the toxic effect of a given neurotoxin preparation on a living organism or an isolated neuromuscular preparation. For establishing the toxic effect in an LD₅₀ assay, the neurotoxin must be biologically active in each of said three activities mentioned above. Moreover, various other assays are available, allowing e.g. to determine whether a neurotoxin or the light chain of the neurotoxin is proteolytically active. Such assays are e.g. based on contacting BoNT/A with SNAP-25. Alternatively, a peptide representing the cleavage site of SNAP-25 can be used, wherein the peptide can be labelled to ease detection. In a preferred aspect, biological activity is determined by using the MPN assay described herein above.

A first polypeptide activated by proteolytically processing an inactive precursor polypeptide, said inactive precursor polypeptide comprising a polypeptide sequence having at least 60% sequence identity with the sequence of SEQ ID NO: 2 is described herein. This rests on the observation that a polypeptide having the polypeptide sequence of SEQ ID NO: 2 is proteolytically inactive, while N-terminal truncations thereof are proteolytically active. Taught herein is the use of the proteolytically inactive polypeptide in the methods described herein. The proteolytically inactive polypeptide described herein can be activated e.g. by removing a fragment from the N-terminus or the entire N-terminus comprising amino acid residues 1 to 248 of SEQ ID NO: 2. In one description, the N-terminus is removed by a protease, in another description, the N-terminus is removed by autoproteolysis of SEQ ID NO: 2. 60% sequence identity refers to a sequence alignment with full length NT02CB1447.

In another aspect, the present invention's method for the manufacture of a proteolytically processed polypeptide comprises the step of purifying the proteolytically processed second polypeptide or at least one or two or more cleavage products thereof. Purification of *C*. *botulinum* expressed BoNT/A may be done e.g. as essentially described in the prior art (DasGupta 1984, Toxicon 22, 415; Sathyamoorthy 1985, J Biol Chemistry 260, 10461). In particular, purification of the neurotoxin can contain one or more precipitation- and extraction steps, one or more concentration steps, and further distinct chromatographic steps. Recombinant single chain BoNT/A and its purification is described in prior art (Rummel et al., 2004, Mol Microbiol. 51:631-43).

In a preferred embodiment, the Clostridium strain is C. botulinum, or a derivative thereof. For fermentation, the process described by DasGupta B. R. et al. in Toxicon, vol. 22, No.3, p. 414 to 424, 1984, can be used. Therefore 0.5% yeast extract and 0.6% autoclaved yeast paste is added to 2% of the N-Z-amine type A medium, and a pH of 7.2 will be adjusted with the help of 4N NaOH, and the medium prepared in such a way will afterwards be autoclaved. To this medium separately autoclaved glucose (20% by weight per volume) may be added, to come to a final concentration of glucose of 0.5% in the medium. Incubation may occur e.g. at 37°C without stirring, wherein the fermentation is discontinued e.g. after 96 hours. It is within the scope of the present invention that besides the batch fermentation described before also semi-batch fermentation, repeated batch fermentation or continuous fermentation can be performed.

After the actual fermentation and separation of the fermentation medium from the cells the fermentation medium may undergo a first precipitation with the goal of removing large proteins. The precipitation is preferably an acid precipitation. Reaction conditions for such an acid precipitation are known to those skilled in the art. Typically 1.5 M H₂SO₄ may be used, to acidify the supernatant to a pH of 3.5. The centrifugation usually occurs for 20 minutes at 2400 x g at 4°C. The pellet received through centrifugation may be washed with water, preferably repeatedly. Subsequently, the pellet may be extracted with a 0.1 M citric acid-trisodium citrate buffer, pH 5.5 e.g. for an hour. Subsequently, a further centrifugation step may be performed, e.g. at 9800 x g for 20 minutes at 4°C. The so obtained pellet optionally can then again be extracted as described before. The supernatant of the extraction, and both supernatants in case of repetition of the extraction, may then be subjected to protamine sulphate precipitation. The precipitation may continue overnight, e.g. at 8°C. Subsequently, the precipitate may be centrifuged, e.g. for 20 minutes at 4°C and at 12,000 x g. The supernatant of centrifugation may be subject to a precipitation such as an ammonium sulphate precipitation, whereby other larger proteins can be removed. After the ammonium sulphate precipitation step another centrifugation step may be added and subsequently the so obtained pellet may be redissolved and, optionally, be subjected to a dialysis. The extract which is preferably dialysed and centrifuged again, can be subjected to a succession of chromatography steps with the objective of purifying the neurotoxin. Each of the chromatography steps serves to remove contaminants such as protamine sulphate, remaining DNA, parts of smaller proteins and middle-sized proteins as well as the hemagglutinins of the botulinum neurotoxin protein complex. For this purpose, one or more chromatography steps may be used in a preferred embodiment. Optionally, the eluate of, e.g. the last chromatography step, may be filtrated in order to reduce germs. Optionally the eluate can be diluted before filtration and suitable adjuvants can be added. During further steps another sterile filtration may be carried out after addition of the adjuvants. In one aspect, the filtration is carried out in reaction containers which may then be subject to a step of lyophilization.

Described herein is a composition obtainable by the present invention's method for the manufacture of a proteolytically processed polypeptide. A composition comprising a mixture of processed and unprocessed second polypeptide, wherein said mixture may contain less than 5%, 4%, 3%, 2% or less than 1% unprocessed second polypeptide is described herein. A composition, wherein said second polypeptide is BoNT or a derivative thereof is described herein. A BoNT e.g. selected from group consisting of BoNT of serotype A, B, C, D, E, F and G, including a derivative thereof is described herein. The composition can be e.g. a liquid or a solid composition and may contain one or more carrier, adjuvants and/or excipients.

A method for the manufacture of a medicament, i.e. a pharmaceutical composition, comprising the steps of the aforementioned method and the further step of formulating the purified di-chain neurotoxin as medicament is described herein. In one description, said medicament comprises a mixture of processed and unprocessed second polypeptide, wherein said mixture contains less than 5% unprocessed second polypeptide. A mixture containing less than 4%, 3%, 2% or less than 1 % unprocessed second polypeptide is described herein.

Various medical uses of the compounds disclosed herein are described:
A proteolytically active polypeptide described herein for use as a medicament or in a pharmaceutical composition.
A composition described herein for use as a medicament or in pharmaceutical composition.
An antibody described herein for use as a medicament or in pharmaceutical composition.
An inhibitor described herein for use as a medicament or in pharmaceutical composition.
A pharmaceutical composition comprising the polypeptide described herein, the antibody described herein, the composition described herein or the inhibitor described herein.

The term "composition" as used herein refers to any composition formulated in solid, liquid, aerosol (or gaseous) form and the like. Said composition comprises e.g. a therapeutically active compound described herein optionally together with suitable auxiliary compounds such as diluents or carriers or further ingredients. In one description, the therapeutically active compound is the proteolytically active polypeptide described herein. A therapeutic compound described herein is the proteolytically processed second polypeptide as described herein above such as the di-chain neurotoxin. Taught herein is a therapeutically active compound which is the antibody described herein. Taught herein is a therapeutically active compound which is the inhibitor of the proteolytically active polypeptide described herein.

In this context, it is distinguished between auxiliary compounds, i.e. compounds which do not contribute to the effects elicited by the compound described herein upon application of the composition for its desired purpose, and further ingredients, i.e. compounds which contribute a further effect or modulate the effect of the compound described herein. Suitable diluents and/or carriers depend on the purpose for which the composition is to be used and the other ingredients. The person skilled in the art can determine such suitable diluents and/or carriers without further ado.

The carrier(s) must be acceptable in the sense of being compatible with the other ingredients of the formulation and being not deleterious to the recipient thereof. The pharmaceutical carrier employed may include a solid, a gel, or a liquid. Exemplary of solid carriers are lactose, terra alba, sucrose, talc, gelatine, agar, pectin, acacia, magnesium stearate, stearic acid and the like. Exemplary of liquid carriers are phosphate buffered saline solution, syrup, oil, water, emulsions, various types of wetting agents, and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania.

The diluent(s) is/are selected so as not to affect the biological activity of the combination. Examples of such diluents are distilled water, physiological saline, Ringer's solutions, dextrose solution, and Hank's solution, in addition, the pharmaceutical composition or formulation may also include other carriers, adjuvants, or non-toxic, non-therapeutic, non-immunogenic stabilisers and the like.

A pharmaceutical composition described herein comprises the biologically active neurotoxin obtained by the method, optionally, one or more pharmaceutically acceptable carrier. The active neurotoxin can be present in liquid or lyophilized form. Said compound can be present together with glycerol, protein stabilisers (e.g., human serum albumin (HSA)) or non-proteinaceous stabilisers such as polyvinylpyrrolidone or hyaluronic acid. The pharmaceutical composition may be administered topically. Conventionally used drug administration is administered intramuscular, subcutaneous (near glands). However, depending on the nature and the mode of action of a compound the pharmaceutical composition may be administered by other routes as well. The di-chain neurotoxin polypeptide is the active ingredient of the composition, and is in one aspect administered in conventional dosage forms prepared by combining the drug with standard pharmaceutical carriers according to conventional procedures. These procedures may involve mixing, granulating, and compression, or dissolving the ingredients as appropriate to the desired preparation. It will be appreciated that the form and character of the pharmaceutical acceptable carrier or diluent is dictated by the amount of active ingredient with which it is to be combined, the route of administration, and other well-known variables.

A therapeutically effective dose refers to an amount of the compound, the neurotoxin, to be used in a pharmaceutical composition described herein which prevents, ameliorates or treats the symptoms accompanying a disease or condition referred to in this specification. Therapeutic efficacy and toxicity of the compound can be determined by standard pharmaceutical procedures in cell cultures or experimental animals, e.g., ED50 (the dose therapeutically effective in 50% of the population) and LD₅₀ (the dose lethal to 50% of the population). The dose ratio between therapeutic and toxic effects is the therapeutic index, and it can be expressed as the ratio, LD₅₀/ED₅₀.

The dosage regimen will be determined by the attending physician and other clinical factors. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment. The pharmaceutical compositions and formulations referred to herein are administered at least once in order to treat or ameliorate or prevent a disease or condition recited in this specification. However, the said pharmaceutical compositions may be administered more than one time.

In one description, the aforementioned composition is a medicament or a cosmetic composition. In one description the said medicament comprising the biologically active neurotoxin can be used for prevention and/or treatment of at least one of the following diseases and disorders: voluntary muscle strength, focal dystonia, including cervical, cranial dystonia, and benign essential blepharospasm, hemifacial spasm, and focal spasticity, gastrointestinal disorders, hyperhidrosis, and cosmetic wrinkle correction, in a further aspect also blepharospasm, oromandibular dystonia, jaw opening type, jaw closing type, bruxism, Meige syndrome, lingual dystonia, apraxia of eyelid, opening cervical dystonia, antecollis, retrocollis, laterocollis, torticollis, pharyngeal dystonia, laryngeal dystonia, spasmodic dysphonia/adductor type, spasmodic dysphonia/abductor type, spasmodic dyspnea, limb dystonia, arm dystonia, task specific dystonia, writer's cramp, musician's cramp, golfer's cramp, leg dystonia, thigh adduction, thigh abduction knee flexion, knee extension, ankle flexion, ankle extension, equinovarus, deformity foot dystonia, striatal toe, toe flexion, toe extension, axial dystonia, pisa syndrome, belly dancer dystonia, segmental dystonia, hemidystonia, generalised dystonia, dystonia in lubag, dystonia in corticobasal degeneration, dystonia in lubag, tardive dystonia, dystonia in spinocerebellar ataxia, dystonia in Parkinson's disease, dystonia in Huntington's disease, dystonia in Hallervorden Spatz disease, dopa-induced dyskinesias/dopa-induced dystonia, tardive dyskinesias/tardive dystonia, paroxysmal dyskinesias/dystonias, kinesiogenic non- kinesiogenic action-induced palatal myoclonus, myoclonus myokymia, rigidity, benign muscle cramps, hereditary chin trembling, paradoxic jaw muscle activity, hemimasticatory spasms, hypertrophic branchial myopathy, maseteric hypertrophy, tibialis anterior hypertrophy, nystagrnus, oscillopsia supranuclear gaze palsy, epilepsia, partialis continua, planning of spasmodic torticollis operation, abductor vocal cord paralysis, recalcitant mutational dysphoria, upper oesophageal sphincter dysfunction, vocal fold granuloma, stuttering Gilles de Ia Tourette syndrome, middle ear myoclonus, protective larynx closure, postlaryngectomy, speech failure, protective ptosis, entropion sphincter Odii dysfunction, pseudoachalasia, nonachalsia, oesophageal motor disorders, vaginistnus, postoperative immobilisation tremor, bladder dysfunction, detrusor sphincter dyssynergia ,bladder sphincter spasm, hemifacial spasm, reinnervation dyskinesias, cosmetic use craw's feet, frowning facial asymmetries, mentalis dimples, stiff person syndrome, tetanus prostate hyperplasia, adipositas, treatment infantile cerebral palsy strabismus, mixed paralytic concomitant, after retinal detachment surgery, after cataract surgery, in aphakia myositic strabismus, myopathic strabismus, dissociated vertical deviation, as an adjunct to strabismus surgery, esotropia, exotropia, achalasia, anal fissures, exocrine gland hyperactivity, Frey syndrome, Crocodile Tears syndrome, hyperhidrosis, axillar palmar plantar rhinorrhea, relative hypersalivation in stroke, in Parkinson's, in amyotrophic lateral sclerosis spastic conditions, in encephalitis and myelitis autoimmune processes, multiple sclerosis, transverse myelitis, Devic syndrome, viral infections, bacterial infections, parasitic infections, fungal infections, in hereditary spastic paraparesis postapoplectic syndrome hemispheric infarction, brainstem infarction, myelon infarction, migraine, in central nervous system trauma, hemispheric lesions, brainstem lesions, myelon lesion, in central nervous system hemorrhage, intracerebral hemorrhage, subarachnoidal hemorrhage, subdural hemorrhage, intraspinal hemorrhage, in neoplasias, hemispheric tumors, brainstem tumors, myelon tumors, snoring (WO 2000/033863). For details and symptoms see, e.g., Jost 2007, Drugs 67(5), 669 or Dressler 2000 in Botulinum Toxin Therapy, Thieme Verlag, Stuttgart, New York.

A composition that is a cosmetic composition which can be formulated as described for a pharmaceutical composition above is described herein. For a cosmetic composition, likewise, it is envisaged that the compound described herein may be used in substantially pure form. Cosmetic compositions may be applied intramuscular. As described herein, cosmetic compositions comprising the neurotoxin can be formulated as an anti-wrinkle solution.

Described herein is a pharmaceutical composition comprising the antibody or the inhibitor described herein. Since the polypeptide described herein is responsible for activating clostridial neurotoxins, the antibody will be useful for reducing the toxic effect observed during infection with clostridia. Therefore, the antibody can be used for treating an infection by Clostridia, including Clostridium perfringens, Clostridium difficile, Clostridium tetani, Clostridium botulinum, Clostridium baratii, Clostridium butyricum, Clostridium sporogenes, Clostridium acetobutylicum, Clostridium haemolyticum, Clostridium novyi and Clostridium oedematiens. Furthermore, the antibody can be used for the treatment of symptoms associated with said infection. Moreover, said antibody can be used in the treatment of a condition or a symptom associated with the condition, wherein the condition is selected from Botulism, Tetanus, Pseudomembranous colitis, Gangrene, Food poisoning and the like.

Described herein is a pharmaceutical composition comprising the proteolytically active polypeptide described herein. Said pharmaceutical composition can be used for proteolytically cleaving polypeptides involved in coaglutination, in particular for treating patients with hypocoaglutination. The pharmaceutical composition can be used as fibrinolyticum, in particular for treating patients with myocard infarct, pulmonary embolism, deep venous thromboembolism, i.e. for removing blood clots. Also envisaged is the use of the pharmaceutical composition in the treatment of stroke. Moreover, the pharmaceutical composition can be used in the treatment of exokrine pancreatic insufficiency for replacing either of trypsin, chemotrypsin, pepsin. Moreover, the pharmaceutical composition can be used in the treatment of patients affected by inflammatory reactions, in the treatment of cancer patients, in particular for proteolytically cleaving surface exposed tumor antigens. Moreover, the pharmaceutical composition can be used in the treatment of papilloma.

A method of screening for an inhibitor comprising the step of (a) contacting the proteolytically active polypeptide described herein with a known substrate and, optionally, with a putative inhibitor; and (b) determining the effect of the putative inhibitor on the conversion of substrate into cleavage product, wherein a reduction in the amount of cleavage product is indicative for the inhibitory effect of the putative inhibitor is described herein. A putative inhibitor which is a peptide comprising an amino acid sequence selected from any one of SEQ ID NOs: 4 to 25, wherein at least one basic amino acid of said amino acid sequence is replaced with a non-basic amino acid is described herein. A peptide comprising one or more chemical modifications is described herein. Any inhibitor which is a peptidomimetic of said peptide is described herein. A putative inhibitor which is part of a chemical compound microarray, i.e. a collection of organic chemical compounds is described herein. Taught herein is an inhibitor which is the antibody described herein. This method is useful for identifying compounds capable of inhibiting the proteolytically active polypeptide described herein. An initial screen may be based on, for example, a peptide comprising an amino acid sequence selected from any one of SEQ ID NOs: 4 to 25. Peptides capable of inhibiting the polypeptide described herein may be modified in order to increase inhibition. Modifications include amino acid substitution or chemical modifications. Typically, this method is carried out by contacting the polypeptide described herein with a known substrate in the presence and absence of a putative inhibitor (step (a) of the method) and by comparing the effect of the putative inhibitor on the conversion of substrate into cleavage product. A reduction of the conversion rate in the presence of putative inhibitor is indicative of an inhibitory effect.

Taught herein is an inhibitor of the proteolytically active polypeptide described herein, wherein said inhibitor is (a) an inhibitor comprising an amino acid sequence as shown in any one of SEQ ID NOs: 4 to 25, wherein a basic amino acid contained therein is replaced with a non-basic amino acid; or (b) the antibody described herein.

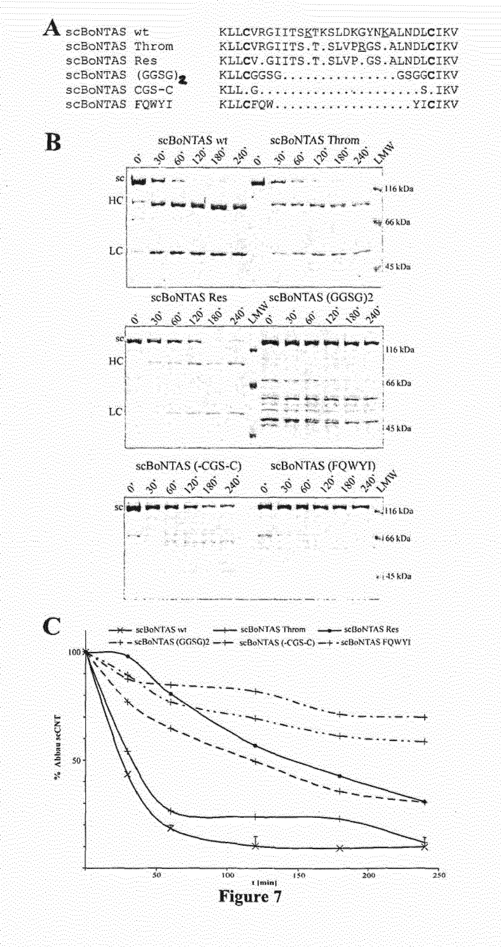

The figures show:
**Figure 1****: Activity test of fractions collected from HiPrep 16/10 Q FF.**
   5 µl of fractions collected from HiPrep 16/10 Q FF run were analysed for enzymatic activity by incubating 2 µg scBoNTA (lane 2) for 1 h at 37°C and subsequent 10% SDS-PAGE. Lane 1: low molecular weight marker (LMW): 116 kDa, 66 kDa, 45 kDa, 35 kDa.
**Figure 2****: Analysis of collected fractions from SEC (HiLoad 16/60 Superdex 75) regarding content of nBH with a molecular weight of -37.3 kDa by 12.5% SDS-PAGE.** Fractions 3 to 11 contain the majority of nBH. (Lane 1: LMW: 116 kDa, 66 kDa, 45 kDa, 35 kDa, 25 kDa, 18.4 kDa, 14.4 kDa)
**Figure 3****: 12.5% SDS-PAGE analysis for determination of purity and protein concentration of three purification batches of nBH.**
   lane 1, LMW (116 kDa, 66 kDa, 45 kDa, 35 kDa, 25 kDa, 18.4 kDa, 14.4 kDa); lane 2, nBH Lot TE311206 (192 ng/µl maturated NT02CB1446/CBO1444, amino acids 254-594 of Genbank acc. CAL82987.1, MW: 38.6 kDa); lane 3, nBH Lot TIK301009 (130 ng/µl maturated NT02CB1447/CBO1445, SEQ ID NO: 1, amino acids 249-581 of Genbank acc. CAL82988.1, MW: 37.3 kDa); lane 4, nBH Lot TIK280509 (114 ng/µl maturated NT02CB1447/CBO1445, SEQ ID NO: 1, amino acids 249-581 of Genbank acc. CAL82988.1, MW: 37.3 kDa).

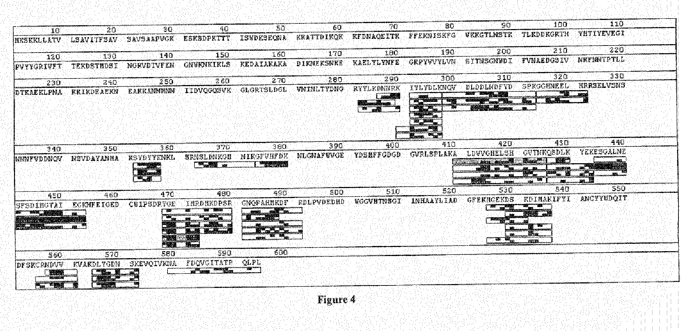

**Figure 4****: ESI-MS/MS spectrum analysis report.**
   38.6 kDa protein band of nBH lot TE311206 was identified as NT02CB1446/CBO1444 with a Mascot score of 725 and a peptide MS/MS sequence coverage of 29.6 % over the entire open reading frame (ORF). No peptide (grey box, identified MS peptide; red squares, identified amino acid y-/b-ion of peptide after MS/MS decay) was identified derived from the N-terminal 253 amino acids. The MS/MS analysis of lot TE311206 displayed a sequence coverage of 52% according to the C-terminal amino acids 254-594 forming the nBH.

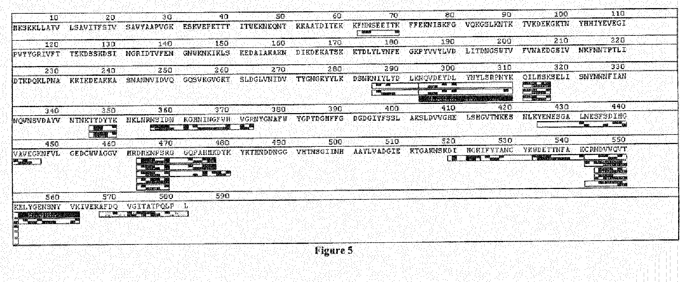

**Figure 5****: ESI-MS/MS spectrum analysis report.**
   37.3 kDa protein band of nBH lot TIK301009 was identified as NT02CB1447/CBO1445 with a Mascot score of 555 and a peptide MSIMS sequence coverage of 28.4 % over the entire open reading frame (ORF). Except one all peptides (grey box, identified MS peptide; red squares, identified amino acid y-/b-ion of peptide after MS/MS decay) identified derive from the C-terminal 333 amino acids. The MS/MS analysis of lot TIK301009 displayed a sequence coverage of 49.5% according to the C-terminal amino acids 249-581 forming the nBH.

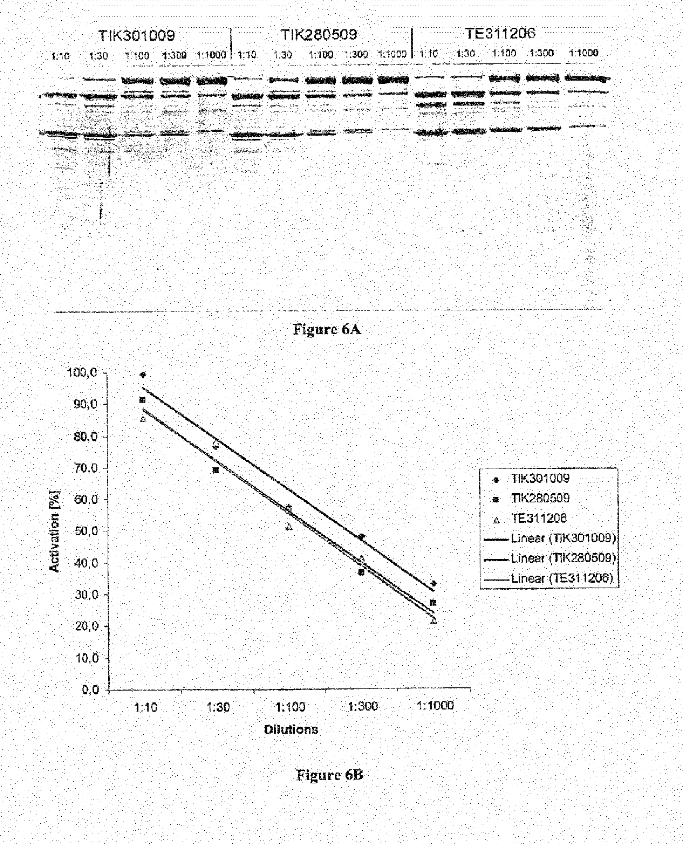

**Figure 6****: Comparison of concentration dependent proteolytic activity of nBH derived from three purification batches.**
   **A** 12.5% SDS-PAGE of activity test analysing nBH derived from the batches TIK301009, TIK280509 and TE311206 using the following dilutions of the concentrated nBH: 1:10, 1:30, 1:100, 1 :300, 1:1000. The assay was performed by incubating 1 µg scBoNT/A and 2 µl dH₂O and 1 µl of correspondingly diluted nBH for 60 min at 37°C. For SDS-PAGE analysis, 3 µl of a reducing 4x SDS Laemmli buffer was added to a final volume of 10 µl. 150 kDa scBoNT/A was cleaved into 100 kDa heavy chain and 50 kDa light chain.
   **B** The optical density of the protein bands of heavy chain, light chain and scBoNT/A were quantified and the sum of light and heavy chain product bands was divided by sum of LC, HC and scBoNT/A protein bands. Higher dilution of first polypeptide decrease cleavage rate. The specific proteolytic activity of the three different batches is nearly identical
**Figure 7****: Time dependent cleavage of scBoNT/A wild-type and mutants containing a modified loop by nBH.**
   A Modification of the loop sequence. In scBoNTAS Throm all lysine residues are removed and the thrombin recognition sequence LVPRGS is inserted whereas in scBoNT Res the loop lacks any basic amino acids. Shortening the loop to eight small residues or five amino acids with bulky side chains yields scBoNTAS (GGSG)₂ and scBoNTAS FQWYI, respectively. In scBoNTAS CGS-C the entire loop is deleted and the disulfide bridge forming cysteines are replaced by glycine and serine.
   **B** SDS-PAGE analysis of time-dependent cleavage of scBoNT/A wild-type and mutants.
   **C** scBoNTAS wild-type is activated by nBH in a time dependent manner into light chain and heavy chain within 120 min. Lack of lysines and insertion of a single arginine residue prolongs the cleavage of the loop (scBoNTAS Throm). A loop lacking any basic residue is still cleavable (scBoNTAS Res). Shortening the loop to 8mer peptide, introducing five amino acids with bulky side chains or deleting the entire loop yields an uncleavable scBoNT/A.

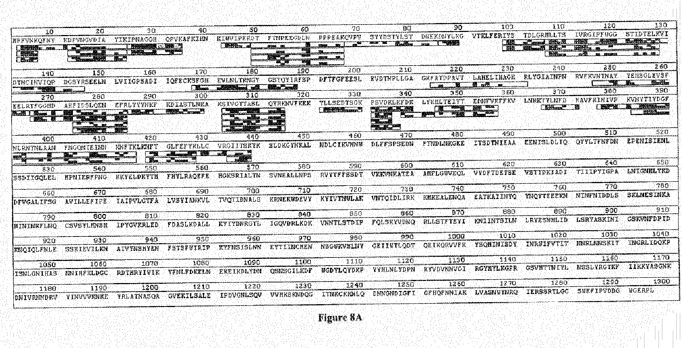

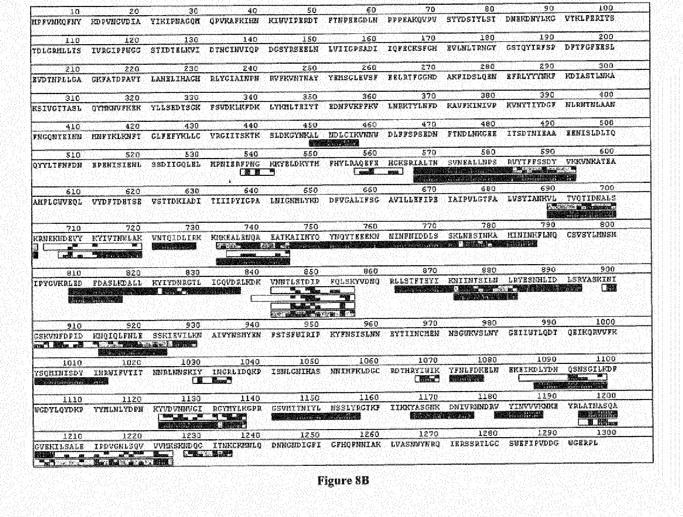

**Figure 8****: MS/MS analysis of the 50 kDa and 100 kDa cleavage products upon digestion of scBoNT/A with nBH.**
   **A** Analysis of the 50 kDa cleavage product which was identified as light chain of BoNT/A with a Mascot score of 1460. The most C-terminal ascribed peptide covers amino acids G433 to K438 which corresponds to the physiologically observed C-terminus of BoNT/A LC.
   **B** Analysis of the 100 kDa cleavage product which was identified as heavy chain of BoNT/A with a Mascot score of 96. The most N-terminal ascribed peptide covers amino acids A449 to K456 which corresponds to the physiologically observed N-terminus of BoNT/A HC.

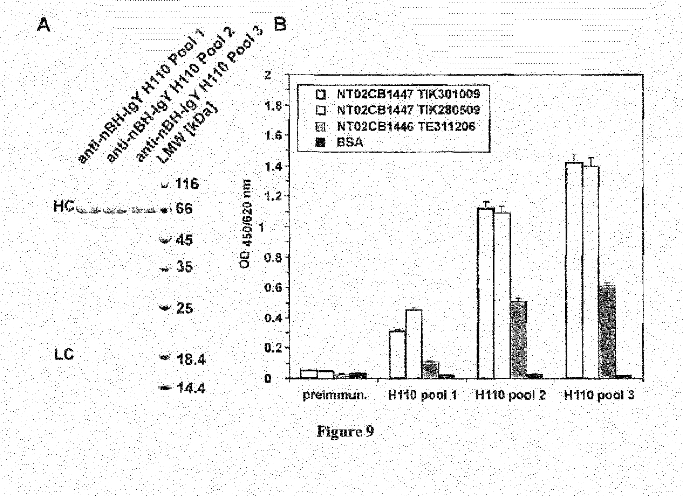

**Figure 9****: A** The protein content (mg/ml) of anti-nBH-IgY of three subsequent pools was analysed by 12.5% SDS-PAGE. **B** ELISA: Nunc Maxisorp F96 microtiter plates were coated with nBH of various lots (500 ng/mL) in PBS overnight at 4°C and then blocked for 1 h with blocking buffer of PBS containing 0.1% Tween-20 and 2% nonfat skimmed milk. After washing, an IgY dilution of each pool (10 µg/ml in blocking buffer) was added for 1 h and detected using biotin-labelled donkey anti-chicken IgY, streptavidin-horseradish peroxidase (both Dianova, Hamburg, Germany) and 3,3',5,5'-tetramethylbenzidine (Sigma).

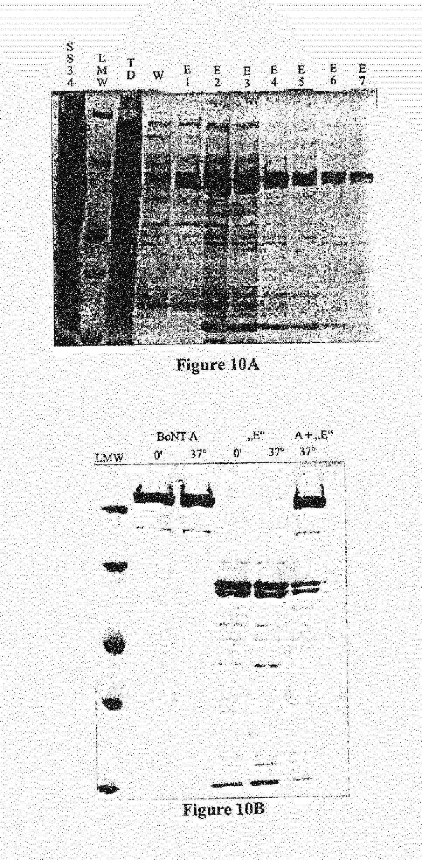

**Figure 10****: A Recombinant expression and isolation of inactive BH 1-581 (63 kDa) by Talon IMAC.** 10% SDS-PAGE analysis of Talon IMAC fractions (LMW: 116 kDa, 66 kDa, 45 kDa, 35 kDa, 25 kDa; SS34, clear lysate; TD, flow through; W, wash fraction; E1-E7, imidazol eluted fractions 1 to 7). **B** No endoproteolyis of scBoNT/A into LC (50 kDa) and HC (100 kDa) with recombinant, iBH (SEQ ID NO: 2; "E"; 63 kDa) is observed at 37°C after 1 h (lane 6)(LMW: 116 kDa, 66 kDa, 45 kDa, 35 kDa, 25 kDa).

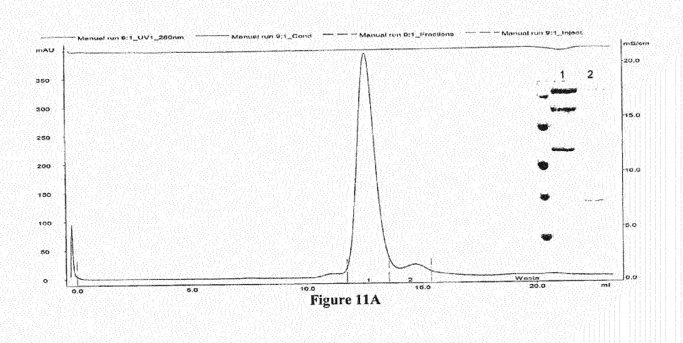

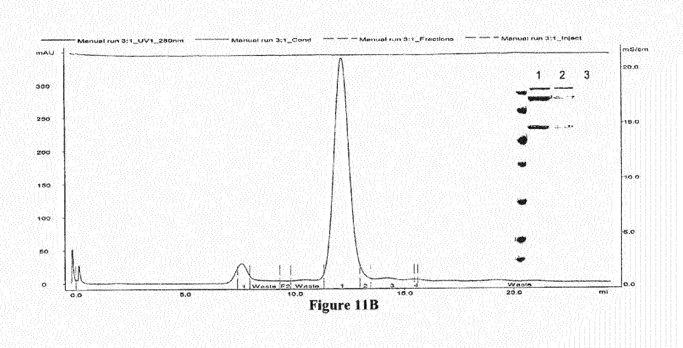

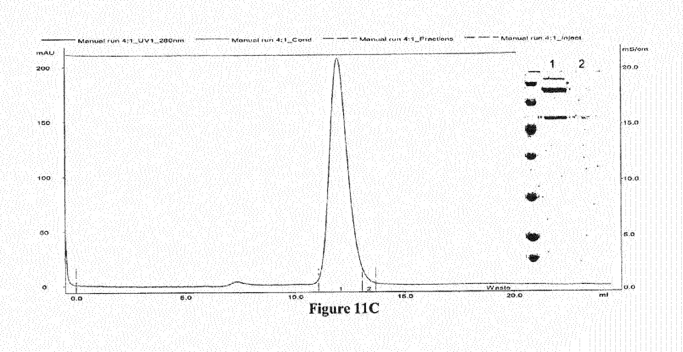

**Figure 11****: Use of purified active BoNTHydrolase (nBH) for obtaining proteolytically processed polypeptide**
   **A** 200 µg of recombinant purified scBoNT/A is incubated with 350 ng purified active BoNTHydrolase for 12 min at 37°C. To stop the reaction nBH is removed by SEC (column Superdex 200 10/300 GL, buffer: 50 mM NaP pH 7.5, 150 mM NaCl, sample volume = 0.3 ml, flow rate = 0.25 ml/min) and the amount of cleavage is analysed by 10% SDS-PAGE. **B** Fraction 1 (1800 µl) containing ∼40% processed BoNT/A is incubated with 350 ng purified active BoNTHydrolase for 15 min at 37°C and concentrated to 300 µl by ultrafiltration. To finally stop the reaction nBH is removed by SEC (column Superdex 200 10/300 GL, buffer: 50 mM NaP pH 7.5, 150 mM NaCl, sample volume = 0.3 ml, flow rate = 0.25 ml/min) and the amount of cleavage is analysed by 10% SDS-PAGE. C Fractions 1 and 2 (1800 µl) containing ~80% processed BoNT/A are combined and incubated with 120 ng purified active BoNTHydrolase for 25 min at 37°C and concentrated to 300 µl by ultrafiltration. To finally stop the reaction nBH is removed by SEC (column Superdex 200 10/300 GL, buffer: 50 mM NaP pH 7.5, 150 mM NaCl, sample volume = 0.3 ml, flow rate = 0.25 ml/min) and the amount of cleavage is analysed by 10% SDS-PAGE. A >95% processed BoNT/A (SEQ ID NO. 3) is obtained.

**The sequence listing shows:**
SEQ ID NO: 1: proteolytically active polypeptide derived from a Clostridium botulinum strain ATCC 3502, GenBank accession No: "CAL82988.1", lacking 248 N-terminal amino acid residues
SEQ ID NO: 2: proteolytically inactive polypeptide derived from a Clostridium botulinum strain ATCC 3502, GenBank accession No: "CAL82988.1"
SEQ ID NO: 3: BoNT/A of ATCC 3502, Genbank acc. "AAA23262"
SEQ ID NO: 4: Loop of BoNT/A1
SEQ ID NO: 5: Loop of BoNT/A2/A6
SEQ ID NO: 6: Loop of BoNT/A3
SEQ ID NO: 7: Loop of BoNT/A3
SEQ ID NO: 8: Loop of BoNT/A4
SEQ ID NO: 9: Loop of BoNT/A5
SEQ ID NO: 10: Loop of BoNT/A7
SEQ ID NO: 11: Loop of BoNT/B1/B4bv/B6
SEQ ID NO: 12: Loop of BoNT/B2/B3
SEQ ID NO: 13: Loop of BoNT/B5np
SEQ ID NO: 14: Loop of BoNT/C/CD
SEQ ID NO: 15: Loop of BoNT/D
SEQ ID NO: 16: Loop of BoNT/DC
SEQ ID NO: 17: Loop of BoNT/E 1 -E5
SEQ ID NO: 18: Loop of BoNT/E6
SEQ ID NO: 19: Loop of BoNT/F1/F6
SEQ ID NO: 20: Loop of BoNT/F2/F3
SEQ ID NO: 21: Loop of BoNT/F4
SEQ ID NO: 22: Loop of BoNT/F5
SEQ ID NO: 23: Loop of BoNT/F7
SEQ ID NO: 24: Loop of BoNT/G
SEQ ID NO: 25: Loop of TeNT
SEQ ID NO: 26: nucleic acid sequence encoding SEQ ID NO: 1
SEQ ID NO: 27: nucleic acid sequence encoding SEQ ID NO: 2

### EXAMPLES

### Example 1: Purification and characterisation of the native BoNTHydrolase (nBH), which specifically cleaves single chain BoNT/A into its active di-chain form

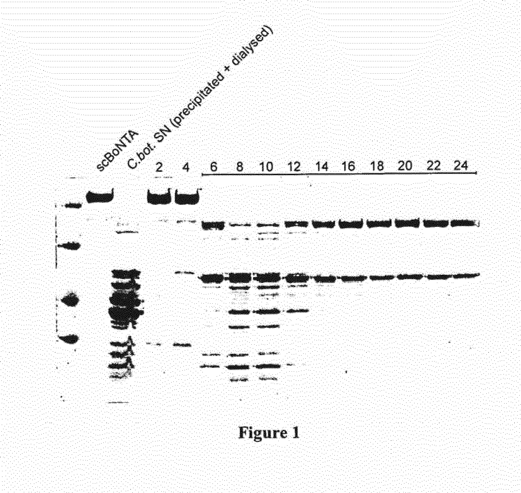

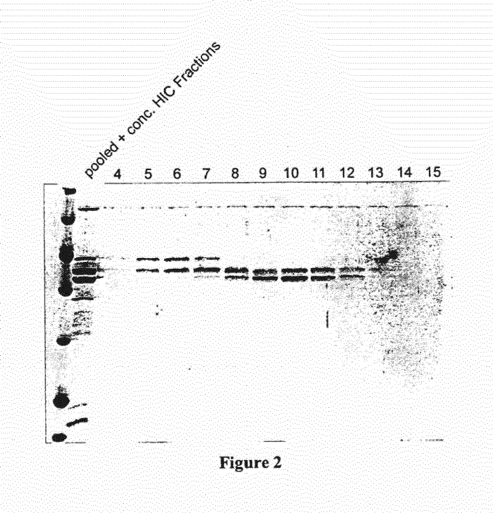

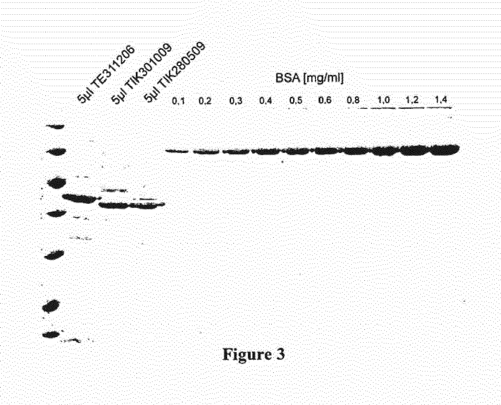

(1) Read-out system/activity test: To specifically detect and purify an enzymatic activity hydrolysing botulinum neurotoxin A (BoNT/A) into the 50 kDa light chain (LC) and 100 kDa heavy chain (HC) in culture supernatants of *C*. *botulinum* and in between chromatographic steps we expressed the 150 kDa BoNT/A as single chain (sc) polypeptide in *E. coli.* Incubation of the recombinant scBoNT/A with the appropriate enzymatic activity (nBH) should yield a 50 kDa LC and a 100 kDa HC visualised by reducing 10-13% SDS-PAGE.
(2) Clostridial protease expression: A single colony of *C*. *botulinum* strain ATCC 3502 was inoculated in 100 ml brain heart infusion (BHI) media and the culture was incubated over night at 37°C under anaerobic conditions. 10 ml of O/N culture was inoculated into I 1 BHI media and anaerobically incubated for 48-72 h.
(3) Ammonium sulphate precipitation: The 1 I culture supernatant was harvested by centrifugation (4°C, 6500xg, 25 min). Ammonium sulphate was added to a final concentration of 85% (here 575 g), the suspension was stirred for 6 hours at 4°C and subsequently centrifuged (4°C, 6500xg, 30 min). The pelleted ammonium sulphate precipitate was dissolved in a small volume (here 5 ml) of 50 mM NaP pH 7.5 and dialysed against 50 mM NaP, 150 mM NaCl pH 7.5. Finally, the dialysate was centrifuged (4°C, 40000xg, 60 min) and the supernatant used for the IEC.
(4) Ion exchange chromatography (IEC, column HiPrep 16/10 Q FF): Supernatant of (3) (Figure 1, lane 3) was applied to a HiPrep 16/10 Q FF anion-exchange column equilibrated and run with a buffer containing 50 mM NaP pH 7.5, 150 mM NaCl. The run was performed at a flow rate of 1 ml/min. An activity test was performed by incubating 5 µl of every other fraction with 2 µg scBoNTA for 1 h at 37°C and subsequent analysis on SDS-PAGE (Figure 1). Fractions 6-24 were combined and its volume concentrated to 3.5 ml by using ultrafiltration (Amicon-Ultra MWCO 10,000).
(5) Size exclusion chromatography (SEC, HiLoad 16/60 Superdex 200): Subsequently, the concentrated protein solution of (4) was loaded on a HiLoad 16/60 Superdex 200 column, equilibrated with 50 mM NaP pH 7,5, 150 mM NaCl. Separation was performed at a flow rate of 1 ml/min. Fractions with a retention volume between 80 ml and 100 ml were analysed using the activity test (1) and the appropriate fractions containing the enzymatic activity (nBH) were combined (~ 10 ml) and concentrated to 3 ml by ultrafiltration. Subsequently, ammonium sulphate was added to a final concentration of 12.5% = 500 mM (+ 0.2 g),
(6) Hydrophobic interaction chromatography (HIC, HiTrap Phenyl Sepharose): nBH was bound to the Phenyl Sepharose in buffer A (50 mM NaP pH 7.5, 500 mM ammonium sulphate). Bound nBH was eluted by reducing the amount of ammonium sulphate due to a linear increasing gradient with buffer B (50 mM NaP pH 7.5) at a flow rate of 1 ml/min. All protein containing fractions were analysed using the activity test (1) and the appropriate fractions were combined and concentrated by ultrafiltration to 3.5 ml. The buffer of the solution was adjusted to 50 mM NaP pH 7.5; 150 mM NaCl.
(7) SEC (HiLoad 16/60 Superdex 75): Finally, the nBH was purified by SEC using the HiLoad 16/60 Superdex 75 column at 50 mM NaP pH 7.5, 150 mM NaCl and a flow rate of 1 ml/min. Fractions with a retention volume between 70 ml and 80 ml were analysed by 12.5% SDS-PAGE (Figure 2) and the fractions 8-12 containing the nBH which migrates at ∼37.3 kDa were combined (~ 10 ml) and concentrated to 1 ml by ultrafiltration.
(8) The prominent protein migrating at approximately 37.3 kDa (nBH) was analysed by N-terminal peptide sequencing according to Edman degradation protocol. The sequence of the identified peptide is V Q G Q S V K G V G and corresponds to the first ten residues of SEQ ID NO: 1.
(9) Two lots of nBH (NT02CB1447, 37.3 kDa, Figure 3, lane 3: TIK301009, lane 4: TIK280509) were reproducibly isolated according to the procedure as described above. Following modifications of the isolation procedure yields the nBH isoform NT02CB1446 (38.6 kDa, Figure 3, lane 2, lot TE311206): (i) growth of *C*. *botulinum* culture: 18 h instead of 48 to 72 h; (ii) variation of chromatographic steps: IEC -> SEC Superdex 75 -> HIC Phenyl Sepharose instead of IEC-> SEC Superdex 200 -> HIC Phenyl Sepharose -> SEC Superdex 75.

### Example 2: Sequence identification of nBH from C. botulinum by mass spectrometry (MS)

(1) Tryptic digestion: The protein bands migrating at approximately 38 kDa (nBH) in SDS-PAGE were excised for tryptic digestion and destained by gently shaking in 50 mM NH₄HCO₃, 50 % acetonitrile for 30 min at 37°C. Destaining was repeated until gel spots were clear. Acetonitrile (100%) was added and removed after 3 min. Subsequently, spots were dried in a speed vac system (Eppendorf, Germany). Trypsin (10 ng/µl) in 50 mM NH₄HCO₃ was added and incubated on ice for 1 h. Then, the remaining trypsin solution was removed, a small volume 50 mM NH₄HCO₃ was added and digestion was carried out at 37°C over night. The supernatant was collected and gel pieces were extracted using 5 % TFA, 10 % acetonitrile for two times. All fluids were combined, dried in a speed vac and extracted peptides were stored at 4°C.

(2) Matrix assisted laser desorption ionisation time of flight (MALDI-TOF/TOF) MS: Samples were analyzed in an MALDI-TOF/TOF mass spectrometer (Ultraflex1 Bruker Daltonik GmbH) in linear mode with an acceleration voltage of 25 kV. Masses were detected from 700 m/z to 4,500 m/z. Samples (2 µl) were cocrystallised with 2 µl of sinnapinic acid solution containing 50 % acetonitril and 0.2 % trifluoric acetic acid (TFA) directly on a stainless steel MALDI target plate. 500 laser shots were collected for each sample.

(3) Peptide separation by reversed phase chromatography: Peptide separation was done by reversed phase chromatography using a nano-HPLC system (Agilent Technologies, Waldbronn, Germany) that consist of an autosampler and a gradient pump. The sample was dissolved in buffer A (5 % acetonitril, 0.1 % formic acid) and an aliquot of up to 10 µl was injected onto a C18 column (Zorbax SB-C18, 5 µm, 300 A, 0.5 mm inner diameter, length 15 cm ) at a flow rate of 5 µl/min. After loading, the column was washed for 15 min with buffer A and the peptides were eluted using a gradient of eluent A and eluent B (70 % (v/v) acetonitrile in 0.1 % (v/v) formic acid) from 0 % to 100 % eluent B in 75 min.

(4) Electrospray ionisation (ESI)-interface and ion trap mass spectrometry: The HPLC outlet was directly connected to the nanoESI source of an ion trap mass spectrometer and the Agilent coaxial sheath-liquid sprayer was used (Agilent Technologies). The outlet capillary was hold by a surrounding steel needle and looked 0.1 to 0.2 mm out of it. The spray was stabilized by N₂ as nebulizer gas (5 1/min). Ionization voltage was set to 4,500 V and dry gas was applied at 5 psi and 250°C. Spectra were collected with an Esquire3000+ ion trap mass spectrometer (Bruker Daltonik) at a scan speed of 13,000 m/z per second. Using ESI in positive mode, mass spectra were acquired from 50 to 1600 m/z in scanning mode and data dependent switching between MS and MS/MS analysis. To increase the quality of MS/MS spectra only two precursor ions from one spectrum were selected for MS/MS analysis and active exclusion was set to 2 min to exclude precursor ions that had already been measured.

(4) Data processing: Data processing was performed with the Data Analysis (version 3.0) and BioTools (version 3.0) software packages (Bruker Daltonik). Protein identification was done using MASCOT software (version 2.1) and MSDB data base (Matrix Science, London, UK).

### (5) Results:

**Table 2: nBH identified by MS**

| **lane** | **Lot nBH** | **Protein concentr.** | **Name of ORF** | **Genbank ace.** | **aa of ORF** | **MW [kDa]** | **Mascot score** |
|---|---|---|---|---|---|---|---|
| 2 | TE311206 | 192 ng/µl | NT02CB 1446 CBO1444 | CAL82987.1 | 254-594 | 38.6 | 725 |
| 3 | TIK301009 | 130 ng/µl | NT02CB1447 CBOl445 | CAL82988.1 | 249-581 | 37.3 | 555 |
| 4 | TIK280509 | 114 ng/µl | NT02CB1447 CBO 1445 | CAL82988.1 | 249-581 | 37.3 | 609 |

The 38.6 kDa protein band of lane 2 (nBH lot TE311206) was identified as NT02CB1446/CB01444 with a Mascot score of 725 and a peptide MSIMS sequence coverage of 29.6 % over the entire open reading frame (ORF). No peptide was identified derived from the N-terminal 253 amino acids (Figure 4). The MS/MS analysis of lot TE311206 displayed a sequence coverage of 52% according to the C-terminal amino acids 254-594 forming the nBH.

The 37.3 kDa protein bands of lane 3 (nBH lot TIK301009) and lane 4 (nBH lot TIK280509) were identified as NT02CB1447/CBO1445 with a Mascot score of 555 and 609, respectively. Except one all peptides identified derive from the C-terminal 333 amino acids (Figure 5). The MS/MS analysis of lot TIK301009 displayed a sequence coverage of 49.5% according to the C-terminal amino acids 249-581 forming the nBH.

### Example 3: Characterisation of enzymatic specificity of nBH

(1) The concentration dependent proteolytic activity of nBH derived from three purification batches was compared (Figure 6). An activity test analysing nBH derived from the batches TIK301009, TIK280509 and TE311206 using various dilutions of nBH demonstrates that higher dilutions decrease the cleavage rate. The proteolytic activity of the three different batches is nearly identical indicating that the maturated isoform NT02CB1446 (TE311206) displays a similar specific activity as the maturated NT02CB1447 (SEQ ID NO: 1).
(2) The time-dependent cleavage of scBoNT/A wild-type and mutants by nBH was analysed employing the activity test (Figure 7). scBoNTAS wild-type is activated by nBH in a time dependent manner into light chain and heavy chain within 120 min by more than 95%. The loop sequence was modified to characterise the cleavage site. In scBoNTAS Throm all lysine residues are removed and the thrombin recognition sequence LVPRGS is inserted which prolonged the cleavage rate. In scBoNT Res the loop lacks any basic amino acids which drastically delays the complete hydrolysis indicating a strong recognition preference of nBH for basic residues like lysine and arginine at the cleavage site. Furthermore, accessibility of nBH to the loop by is impaired by shortening the loop to eight small residues or five amino acids with bulky side chains (scBoNTAS (GGSG)₂ and scBoNTAS FQWYI).
(3) The MS/MS analysis of the 50 kDa cleavage product upon digestion of scBoNT/A with nBH exhibited that the most C-terminal ascribed peptide covers amino acids G433 to K438 which corresponds to the physiologically observed C-terminus of BoNT/A LC (Figure 8A). Analysis of the 100 kDa cleavage product which was identified as heavy chain of BoNT/A demonstrated that the most N-terminal ascribed peptide covers amino acids A449 to K456 which corresponds to the physiologically observed N-terminus of BoNT/A HC (Figure 8B). Thus, the isolated nBH yields physiologically processed BoNT/A and preferentially hydrolyses peptide bonds C-terminal to lysine and arginine residues.

### Example 4: Evolutionary conservation of BoNTHydrolase and its isoforms

Protein sequence analysis of SEQ ID NO: 2 (Genbank acc. CAL82988.1/YP_001253958.1) revealed three conserved domains. Residues 18-573 correspond to a Zinc metalloprotease (elastase) or LasB involved in amino acid transport and metabolism with a Blast score of 738. Residues 148-212 correspond to a peptidase propeptide and YPEB domain or PepSY (Blast score 97. Residues 336-573 are part of the peptidase M4 family including thermolysin, protealysin, aureolysin and neutral proteases (Blast score 803).

The genome sequencing of *C*. *botulinum* ATCC 3502 has revealed the existence of six ORFs encoding iBH isoforms (Sebaihia et al., 2007, Genome Res. 17(7):1082-1092). Further genome data is available for 10 group I *C*. *botulinum* strains as well as the non-BoNT secreting *C*. *sporogenes* which all contain between five to seven ORFs encoding iBH. The nBH (SEQ ID NO: 1) shares minimum 64 % amino acid sequence identity with the other 63 isoforms.

### Example 5: Generation of antibodies specific for the BoNTHydrolase

(1) Generation of IgY: Sixteen-week-old chickens [ISA Brown and Lohmann Selected Leghorn (LSL), Spreenhagener Vermehrungsbetrieb für Legehennen GmbH, Bestensee, Germany] were kept in individual cages, exclusively constructed for the maintenance of chickens (Ebeco, Castrop-Rauxel, Germany). Food (ssniff Legehühner-Zucht 1 and 2; ssniff Spezialitäten GmbH, Soest, Germany) and water were available *ad libitum*, and the chickens started laying eggs between 23 and 25 weeks of age. Eggs were collected daily, labelled, and stored at 4°C until they were further processed. All animal maintenance and experiments were performed in accordance with the guidelines of local authorities, Berlin (No. H0069/03). Chickens were immunized and boosted via the i.m. route (pectoralis muscle, left and right side) a total of 10 times over a 1-year period, with intervals between 4 and 8 weeks. The interval used was based on previous work that showed no demonstrable memory cells until at least 3 weeks postimmunisation (Pei and Collisson, 2005). The antigen concentration used was approximately 20 µg per injection (nBH). No more than 500 µl of antigen solution was injected per immunization. Freund's complete adjuvant was used for the first immunisation, and FIA was used for the subsequent booster injections. The method for IgY purification was adapted from Polson et al. (1980). Briefly, the egg yolk was diluted 1:2 with sterile PBS (pH 7.4, Roche, Mannheim, Germany). For elimination of lipids and lipoprotein, 3.5% (w/v) polyethylene glycol (PEG) 6000 (Roth, Karlsruhe, Germany) was added. After gentle shaking followed by centrifugation (10,000xg for 20 min at 4°C), the supernatant was decanted and solid PEG 6000 was added to a final concentration of 12% (w/v). This mixture was then centrifuged as above. The precipitate was dissolved in 10 ml of PBS, PEG was added to 12% (wt/vol), and the solution was centrifuged. Finally, the precipitate was dissolved in 1.2 ml of PBS, transferred into a microdialysis device (QuixSep, Roth, Germany) and dialysed against PBS at 4°C. The protein content (mg/ml) was analysed by 12.5% SDS-PAGE (Figure 9A) and measured photometrically at 280 nm and was calculated according to the Lambert-Beer law with an extinction coefficient of 1.33 for IgY.
(2) ELISA: Nunc Maxisorp F96 microtiter plates (VWR International GmbH, Darmstadt, Germany) were coated with nBH of various lots (500 ng/mL) in PBS overnight at 4°C and then blocked for 1 h with blocking buffer of PBS containing 0.1% Tween-20 and 2% nonfat skimmed milk (Merck, Darmstadt, Germany). After washing, a IgY dilution (10 µg/ml in blocking buffer) was added for 1 h and detected using biotin-labeled donkey antichicken IgY, streptavidin-horseradish peroxidase (both Dianova, Hamburg, Germany) and 3,3',5,5'-tetramethylbenzidine (Sigma). Detected nBH is illustrated in Figure 9B.
(3) Western blot: nBH was separated per 12.5% SDS-PAGE, and were transferred onto a polyvinylidene fluoride membrane (Invitrogen GmbH, Karlsruhe, Germany) using standard immunoblotting techniques. The membrane was blocked overnight at 4°C, and incubated with IgY (1:5,000 in blocking buffer) for 1 h. After washing, the membrane was probed with biotin-labelled donkey anti-chicken IgY for 30 min and was developed using alkaline phosphatase and CDP-Star (Perkin Elmer, Waltham, MA).

### Example 6: Recombinant expression of BoNTHydrolase

(1) Plasmid constructions: The gene portions encoding native BH (SEQ ID NO: 1) and its propeptide (SEQ ID NO: 2) were amplified by PCR using suitable oligonucleotides and genomic DNA of C. botulinum ATCC 3502, fused to an oligonucleotide coding for the His6Tag and inserted into pQE3 (Qiagen) yielding the expression plasmid pQ-BH1445H6-249-581 and pQ-BH1445H6-1-581, respectively. Nucleotide sequences were verified by DNA sequencing.
(2) Purification of recombinant proteins: nBH and iBH, fused to a carboxyl-terminal His6Tag, were produced utilizing the *E*. *coli* strain M 1 5pREP4 (Qiagen) during ten hours of incubation at room temperature, and were purified on Talon-sepharose beads (Clontech Inc.) following to the manufacturer's instructions. Fractions containing the desired proteins were pooled, frozen in liquid nitrogen, and kept at -70°C. iBH was isolated as recombinant protein with a MW of 63 kDa (Figure 10A). The inactivity of iBH was demonstrated using the activity test: after 1 h at 37°C no scBoNT/A wt was hydrolysed in LC and HC (Figure 10B).

### Example 7: Inhibition of BoNTHydrolase

(1) Screening for peptide inhibitors of BH: Peptides based on SEQ ID NOs: 4 to 25 will be synthesised lacking one or more basic residues. Each peptide will be added to the mixture according to the activity test. A peptide being able to decrease the amount of processed scBoNT/A, prolong the duration required for fully processing scBoNT/A or block processing scBoNT/A is considered to be an inhibitor of nBH.
(2) Screening for antibody-based inhibitors: Antibodies generated against epitopes derived from nBH like IgY of Example 5 are incubated with nBH and subsequently subjected to the activity test. An antibody being able to decrease the amount of processed scBoNT/A, prolong the duration required for fully processing scBoNT/A or block processing scBoNT/A is considered to be an inhibitor of nBH.

### Example 8: Use of purified active BoNTHydrolase (nBH) for obtaining proteolytically processed polypeptide

(1) 200 µg of recombinant purified scBoNT/A is incubated with 350 ng purified active BoNTHydrolase for 12 min at 37°C. To stop the reaction nBH is removed by SEC (column Superdex 200 10/300 GL, buffer: 50 mM NaP pH 7.5, 150 mM NaCl, sample volume = 0.3 ml, flow rate = 0.25 ml/min) and the amount of cleavage is analysed by 10% SDS-PAGE (Figure HA).

(2) Fraction 1 (1800 µl) containing ~40% processed BoNT/A is incubated with 350 ng purified active BoNTHydrolase for 15 min at 37°C and concentrated to 300 µl by ultrafiltration. To finally stop the reaction nBH is removed by SEC (column Superdex 200 10/300 GL, buffer: 50 mM NaP pH 7.5, 150 mM NaCl, sample volume = 0.3 ml, flow rate = 0.25 ml/min) and the amount of cleavage is analysed by 10% SDS-PAGE (Figure 11B).

(2) Fractions 1 and 2 (1800 µl) containing ~80% processed BoNT/A are combined and incubated with 120 ng purified active BoNTHydrolase for 25 min at 37°C and concentrated to 300 µl by ultrafiltration. To finally stop the reaction nBH is removed by SEC (column Superdex 200 10/300 GL, buffer: 50 mM NaP pH 7.5, 150 mM NaCl, sample volume = 0.3 ml, flow rate = 0.25 ml/min) and the amount of cleavage is analysed by 10% SDS-PAGE (Figure 11C). A >95% processed BoNT/A (Seq ID NO. 3) is obtained. The identical fully processed second polypeptide (>95% processed BoNT/A) is obtained if the second polypeptide is processed in one step for 50 min at 37°C (200 µg scBoNT/A incubated with 350 ng nBH). After an incubation time of 1h at 37°C, more than 97% of BoNT/A is processed.

## Claims

1. Use of Lys-C or Lys-N for proteolytically processing a single-chain botulinum neurotoxin serotype F (BoNT/F) by hydrolysis to produce an active di-chain BoNT/F.

2. Use according to claim 1, wherein the single-chain BoNT/F is a naturally occurring neurotoxin, a recombinant neurotoxin, or modified neurotoxin, such as a neurotoxin lacking the native H_{c} domain or parts thereof or derivatives with other amino acid residues replacing the neurotoxin H_{c} domain.

3. Method for the manufacture of a proteolytically processed polypeptide, comprising the step of contacting:
(a) a first polypeptide, said first polypeptide being Lys-C or Lys-N;
with
(b) a second polypeptide, said second polypeptide being susceptible to proteolysis by said first polypeptide;
wherein said contacting results in proteolytic processing of said second polypeptide into at least two cleavage products;
wherein the second polypeptide is a single-chain BoNT/F and wherein said first polypeptide hydrolyses the single-chain BoNT/F to produce an active di-chain BoNT/F.

4. The method according to claim 3, wherein said single-chain BoNT/F is a naturally occurring neurotoxin, a recombinant neurotoxin, or modified neurotoxin, such as a neurotoxin lacking the native H_{c} domain or parts thereof or derivatives with other amino acid residues replacing the neurotoxin H_{c} domain.

5. The method according to claim 3 or 4, wherein said contacting occurs within a cell, in a cell lysate, or in a purified cell lysate.

## Patentansprüche

1. Verwendung von Lys-C oder Lys-N zur proteolytischen Verarbeitung eines einkettigen Botulinum-Neurotoxin-Serotyps F (BoNT/F) durch Hydrolyse, um einen aktiven zweikettigen BoNT/F zu produzieren.

2. Verwendung nach Anspruch 1, wobei der einkettige BoNT/F ein natürlich vorkommendes Neurotoxin, ein rekombinantes Neurotoxin oder ein modifiziertes Neurotoxin ist, wie ein Neurotoxin, dem die native H_{c}-Domäne oder Teile davon fehlen, oder Derivate mit anderen Aminosäureresten, die die Neurotoxin-H_{c}-Domäne ersetzen.

3. Verfahren zur Herstellung eines proteolytisch verarbeiteten Polypeptids, umfassend den Schritt eines Inkontaktbringens:
(a) eines ersten Polypeptids, wobei das besagte erste Polypeptid Lys-C oder Lys-N ist;
mit
(b) einem zweiten Polypeptid, wobei das besagte zweite Polypeptid für eine Proteolyse durch das besagte erste Polypeptid empfänglich ist;
wobei das besagte Inkontaktbringen zu einer proteolytischen Verarbeitung des besagten zweiten Polypeptids zu mindestens zwei Spaltprodukten führt;
wobei das zweite Polypeptid ein einkettiger BoNT/F ist und wobei das besagte erste Polypeptid den einkettigen BoNT/F hydrolysiert, um einen aktiven zweikettigen BoNT/F zu produzieren.

4. Verfahren nach Anspruch 3, wobei der besagte einkettige BoNT/F ein natürlich vorkommendes Neurotoxin, ein rekombinantes Neurotoxin oder ein modifiziertes Neurotoxin ist, wie ein Neurotoxin, dem die native H_{c}-Domäne oder Teile davon fehlen, oder Derivate mit anderen Aminosäureresten, die die Neurotoxin-H_{c}-Domäne ersetzen.

5. Verfahren nach Anspruch 3 oder 4, wobei das besagte Inkontaktbringen innerhalb einer Zelle, in einem Zelllysat oder in einem aufgereinigten Zelllysat erfolgt.

## Revendications

1. Utilisation de Lys-C ou de Lys-N pour le traitement protéolytique d'une neurotoxine botulique de sérotype F à simple chaîne (BoNT/F) par hydrolyse pour produire une BoNT/F à double chaîne active.

2. Utilisation selon la revendication 1, dans lequel la BoNT/F à simple chaîne est une neurotoxine d'origine naturelle, une neurotoxine recombinante, ou une neurotoxine modifiée, telle qu'une neurotoxine dépourvue de domaine H_{c} natif ou de parties de celui-ci ou des dérivés avec d'autres résidus d'acide aminé remplaçant le domaine H_{c} de neurotoxine.

3. Procédé pour la fabrication d'un polypeptide traité de manière protéolytique, comprenant l'étape de mise en contact :
(a) d'un polypeptide, ledit premier polypeptide étant Lys-C ou Lys-N ;
avec
(b) un deuxième polypeptide, ledit deuxième polypeptide étant prédisposé à une protéolyse par ledit premier polypeptide ;
dans lequel ladite mise en contact a pour résultat le traitement protéolytique dudit deuxième polypeptide en au moins deux produits de clivage ;
dans lequel le deuxième polypeptide est une BoNT/F à simple chaîne et dans lequel le premier polypeptide hydrolyse la BoNT/F à simple chaîne pour produire une BoNT/F à double chaîne active.

4. Procédé selon la revendication 3, dans lequel ladite BoNT/F à simple chaîne est une neurotoxine d'origine naturelle, une neurotoxine recombinante, ou une neurotoxine modifiée, telle qu'une neurotoxine dépourvue de domaine H_{c} natif ou de parties de celui-ci ou des dérivés avec d'autres résidus d'acide aminé remplaçant le domaine H_{c} de neurotoxine.

5. Procédé selon la revendication 3 ou 4, dans lequel ladite mise en contact a lieu au sein d'une cellule, dans un lysat cellulaire, ou dans un lysat cellulaire purifié.
